(19) Europäisches Patentamt
European Patent Office
Office européen des brevets

(11) Publication number: **0 456 183 B1**

(12) **EUROPEAN PATENT SPECIFICATION**

(45) Date of publication of patent specification: **04.10.95**

(51) Int. Cl.[6]: **C07D 209/96**, A61K 31/395, C07D 205/12, C07D 221/20

(21) Application number: **91107396.3**

(22) Date of filing: **07.05.91**

(54) **Spiro dibenzosuberane derivatives.**

(30) Priority: **07.05.90 JP 118126/90**

(43) Date of publication of application:
**13.11.91 Bulletin 91/46**

(45) Publication of the grant of the patent:
**04.10.95 Bulletin 95/40**

(84) Designated Contracting States:
**AT BE CH DE DK ES FR GB GR IT LI LU NL SE**

(56) References cited:
**EP-A- 0 303 512**

**JOURNAL OF MEDICINAL CHEMISTRY, vol. 7, no. 4, 1964, Columbus, Ohio, US, pages 439-445; M.A. DAVIS et al.: "Anticonvulsants. II. Spirocompounds. Dibenzo[a,d]cyclohepatdiene-5,5'-hydantoin-s, -5,5'-oxazolidinediones, and -5,2'-succinimides"**

(73) Proprietor: **SHIONOGI SEIYAKU KABUSHIKI KAISHA**
**1-8, Doshomachi 3-chome,**
**Chuo-ku**
**Osaka 541 (JP)**

(72) Inventor: **Matsumura, Hiromu**
**15-10-839, Asahigaoka-cho**
**Ashiya-shi,**
**Hyogo (JP)**
Inventor: **Adachi, Makoto**
**6-18-22, Midorigaoka**
**Heguri-cho,**
**Ikoma-gun,**
**Nara (JP)**
Inventor: **Yano, Toshisada**
**5-19-18, Kitaochiai**
**Suma-ku,**
**Kobe-shi,**
**Hyogo (JP)**
Inventor: **Hashizume, Hiroshi**
**1-1-1118, Ikeda-cho**
**Kita-ku,**
**Osaka-shi,**
**Osaka (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

**JOURNAL OF MEDICINAL CHEMISTRY, vol. 33, no. 3, 1990, Columbus, Ohio, US, pages 1069-1076; J.A. MONN et al.: "Synthesis and structure-activity relationship of C5-substituted analogues of (+/-)10,11-dihydro-5H-dibenzo[a,d]cyclohept-en-5,10-imine[(+/-)-desmethyl-MK801]: ligands for the NMDA receptor-coupled phencyclidine binding site"**

Inventor: **Eigyo, Masami**
**2-10-7, Shikanodainishi**
**Ikoma-shi,**
**Nara (JP)**
Inventor: **Matsushita, Akira**
**2-3-22, Fukaeminami-machi**
**Higashinada-ku,**
**Kobe-shi,**
**Hyogo (JP)**

(74) Representative: **VOSSIUS & PARTNER**
**Postfach 86 07 67**
**D-81634 München (DE)**

## Description

This invention relates to novel spiro dibenzosuberane derivatives with antineurodegenerative activities.

Recently, the population has been aging, and the frequency of the ischemic encephalopathy has increased. Therefore, the ischemic encephalopathy and Alzheimer's disease are now social problems. It is suggested that these diseases are caused by the disorder of receptors and neurotransmitters in the cerebral cortex. And the disorder is thought to be mediated with excitatory amino acids such as glutamate and aspartate which act on N-methyl-D-aspartate (NMDA) receptor.

Accordingly, NMDA antagonists competing with the neurotransmitter at the NMDA receptors competitively or non-competitively are effective against the disease, and such agents have been investigated and developed. However, no agents with excellent activity have been marketed.

It is the object of the present invention to provide novel spiro dibenzosuberane derivatives with antineurodegenerative activities. These compounds are noncompetitive antagonists for the NMDA receptors, whereby they are thought effective for the protection against ischemic encephalopathy, the prevention of necrosis of brain cells, and the prevention or treatment for Alzheimer's disease.

In J. Med. Chem., 7 (1964) 439-445 spiro compounds are disclosed which have anticonvulsant properties.

J. Med. Chem. 33 (1990) 1069-1076 discloses $C_5$-substituted analogues of (±)-10,11-Dihydro-5H-dibenzo-[a,d]cyclohepten-5,10-iminewhich are ligands for the NMDA receptor-coupled phencyclidine binding site.

EP-A- 303 512 describes substituted dibenzocycloheptenimines which are useful as anticonvulsant agents and in the treatment of neurodegenerative diseases.

The present invention relates to the compounds of the formula (I):

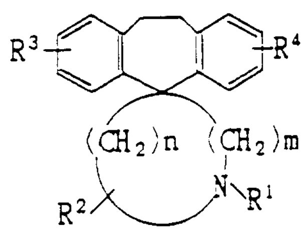

( I )

wherein $R^1$ is hydrogen, alkyl, alkenyl, or aralkyl; $R^2$ is hydrogen or alkyl; $R^3$ and $R^4$ which are the same or different are hydrogen, alkyl, alkoxy, or halogen; m is 0 or 1; n is an integer of 1-4 or a pharmaceutically acceptable acid addition salt thereof, excluding spiro(dibenzosuberane-5,3'-piperidine).

In this specification, the term "alkyl" refers to a straight or branched chain $C_1$ to $C_5$ alkyl, including e.g. methyl, ethyl, n-propyl, isopropyl, n-butyl, isobutyl, sec-butyl, tert-butyl, n-pentyl, isopentyl, neopentyl, tert-pentyl, 2-methylbutyl, n-hexyl and isohexyl.

The term "alkenyl" refers to $C_{1-4}$ alkenyl, including e.g. vinyl, allyl, propenyl and isopropenyl.

The term "aralkyl" refers to $C_{1-4}$ substituted phenyl or naphthyl, including e.g. benzyl, phenethyl, phenylpropyl, naphthylmethyl.

The term "alkoxy" refers to $C_1$-$C_5$ alkoxy.

The term "halogen" refers to fluorine, chlorine, bromine, and iodine.

Pharmaceutically acceptable acid addition salt includes mineral acid salts such as hydrochloride sulfate, perchlorate, nitrate, and phosphate, and organic acid salts such as acetate, oxalate, succinate, maleate, and fumarate.

The compounds of this invention can be prepared by the following methods.

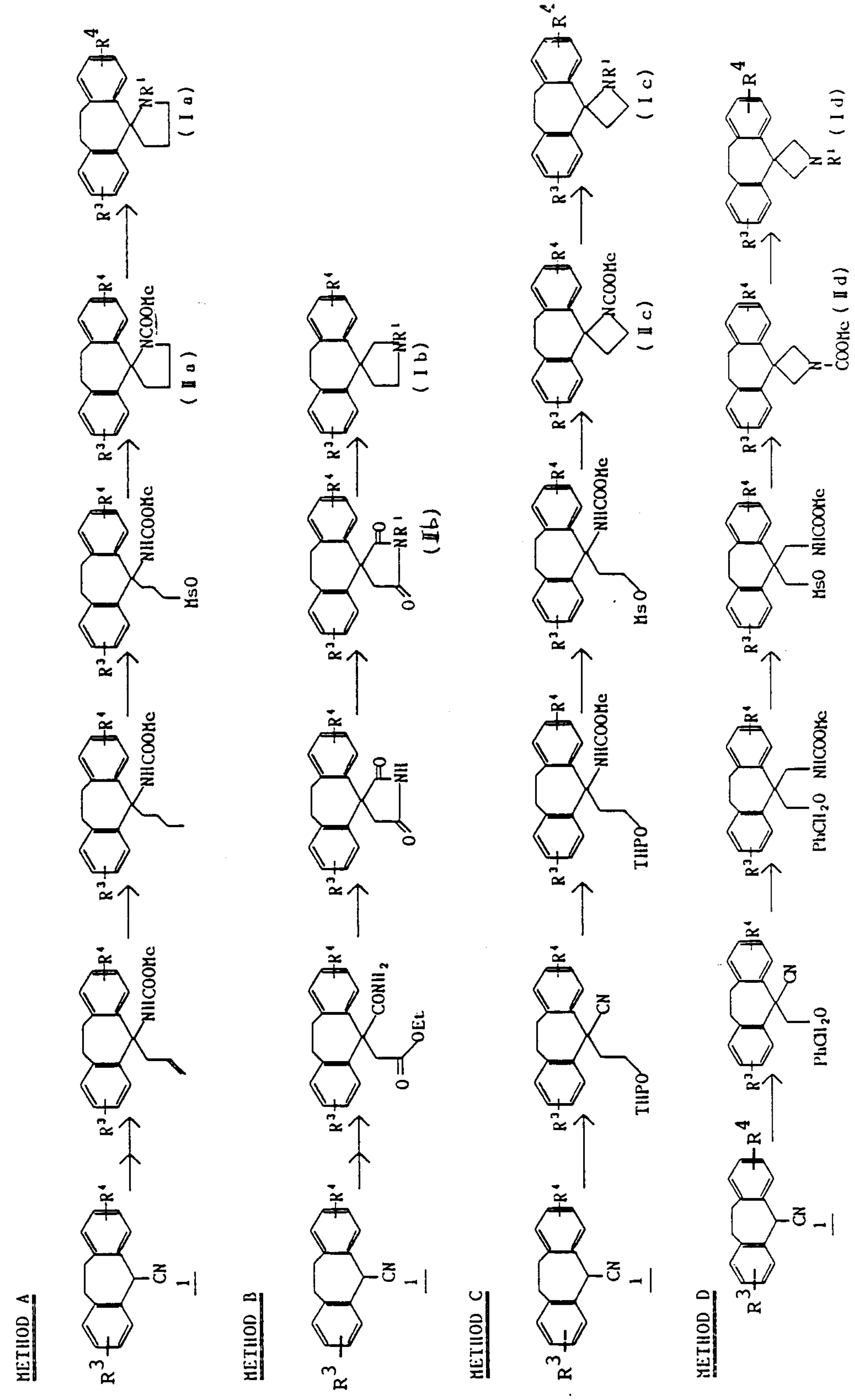

METHOD E

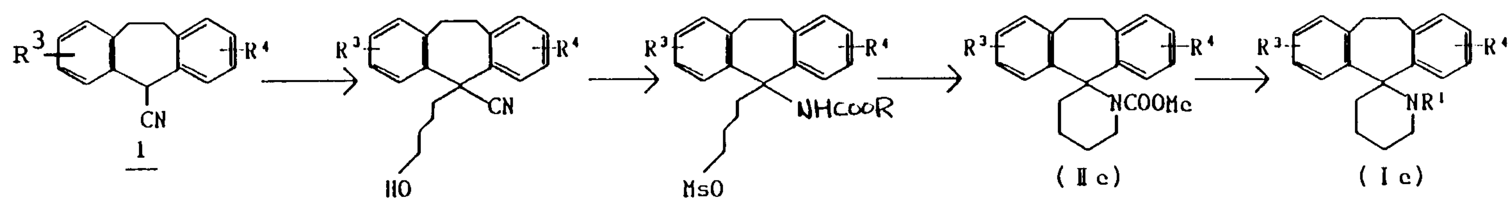


1 (IIc) (Ic)

METHOD F

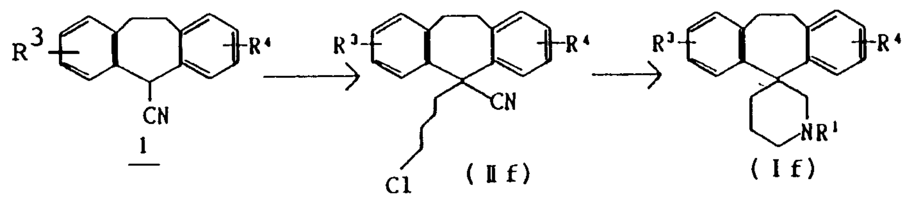

1 (IIf) (If)

METHOD G

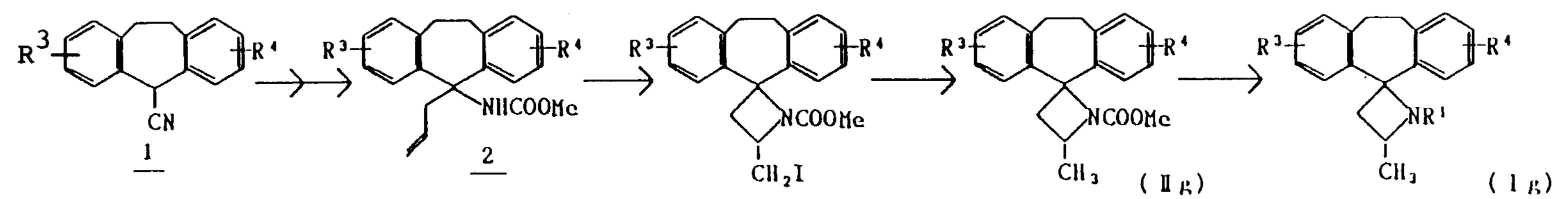

1 2 (IIg) (Ig)

METHOD H

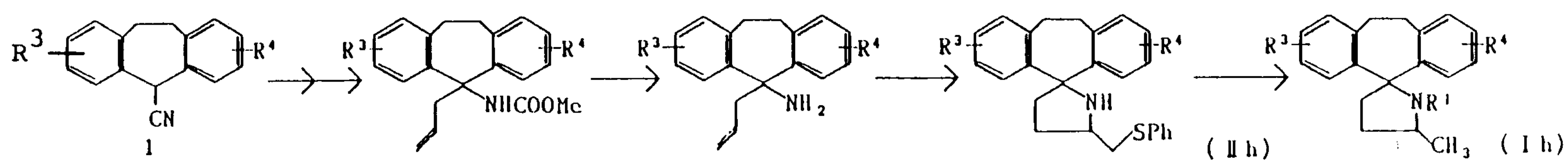

1 (IIh) (Ih)

Wherein $R^1$, $R^2$, $R^3$, and $R^4$ each has the same meaning as defined above.

## METHOD A

The compound (IIa) prepared from 5-cyanodibenzosuberane (1) through 5 steps.

(i) When $R^1 = Me$,

the compound (IIa) is subjected to reduction in an appropriate solvent at room temperature or under heating to give the compound (I a).

As appropriate solvents which may be used are ethers such as diethyl ether and tetrahydrofuran.

As reductants which may be used are lithium aluminium hydride, diborane and the like, most preferably lithium aluminium hydride.

(ii) When $R^1 \neq Me$,

the compound (II a) is refluxed under heating in the presence of a base such as potassium hydroxide and sodium hydroxide in n-butanol, and subjected to hydrolysis, then treated with an acid such as hydrochloric or sulfuric acid to decarboxylation. Then if necessary, the obtained compound is reacted to introduce a group $R^1$ which is different from Me ($CH_3$), e.g. with an alkyl halide, an allyl halide or benzylbromide, or with an alkyl sulfate such as dimethyl sulfate and diethyl sulfate in an inactive solvent such as acetonitrile, dimethylformamide, benzene and toluene under heating, or reacted with an acyl halide such as acetylchloride and propionyl chloride in the presence of a base such as triethylamine and pyridine, and treated with lithium aluminium hydride to prepare the compound (Ia).

## METHOD B

The compound (II b) is prepared from 5-cyanodibenzosuberane (1) through 4 steps. Then the compound (II b) is subjected to reduction in the presence of lithium aluminium chloride in an inactive solvent at room temperature or under heating to give the compound (I b).

As inactive solvents which may be used are ethers such as diethylether and tetrahydrofuran.

If necessary, the compound (I b) is subjected to dealkylation. The compound (I b) is reacted with α-chloroethyl chloroformate in ethylene dichloride and mixed with methanol, and the reaction mixture is treated at 10-90 °C, preferably at 30-40 °C.

## METHOD C

The compound (II c) is prepared from the compound 1 through 6 steps.

(i) When $R^1 = Me$,

the compound (II c) is subjected to reduction in an organic solvent at room temperature or under heating, to give the compound (I c).

The same organic solvent and reductant as in METHOD A may be used.

(ii) When $R^1 \neq Me$,

the compound (II c) is refluxed in n-butanol in the presence of the base such as potassium hydroxide and sodium hydroxide under heating, and subjected to hydrolysis then treated with an acid such as hydrochloride and sulfuric acid to decarboxylation. If necessary, the obtained compound is reacted according to method A (ii) e.g. with an alkyl halide an allyl halide or benzyl bromide, or with an alkyl sulfate such as dimethyl sulfate and diethyl sulfate in an inactive solvent such as acetonitrile, dimethyl formamide, benzene, and toluene under heating, or reacted with an acyl halide such as acetylchloride and propionyl chloride in the presence of a base such as triethylamine and pyridine, and treated with lithium aluminium hydride to prepare the compound (I c).

## METHOD D

The compound (II d) is prepared from the compound 1 through 6 steps.

(i) When $R^1 = Me$,

the compound (II d) is subjected to reduction in an appropriate solvent at room temperature or under heating to give the compound ( I d).

The reaction may be performed in the same condition as METHOD A (i).

(ii) When $R^1 \neq Me$,

the compound (II d) is subjected to decarboxylation, if necessary subjected to $R^1$ introduction, e.g. alkylation, to give the compound (Id).

The reaction may be performed in the same condition as METHOD A (ii).

## METHOD E

The compound (II e) is prepared from the compound 1 through 5 steps.

(i) When $R^1 = Me$,

(ii) When $R^1 \neq Me$,

in each case of (i) and (ii), the reaction may be performed in the same manner as METHOD A to give the compound (I e).

## METHOD F

At first, chloropropyl group is introduced to the compound 1 to give the compound (II f).

The compound 1 is reacted with chloropropyl bromide in the presence of a base such as sodium hydride and potassium tert-butoxide in an inactive solvent such as dimethylformamide, dioxane, and tetrahydrofuran under nitrogen atmosphere under cooling or at room temperature to give the compound (II f). Then the compound (II f) is subjected to reduction and ring closure to give the compound (I f).

The compound (II f) is treated with lithium aluminium hydride in the presence of aluminium chloride in ether, and reacted with potassium carbonate in dimethylformamide.

If necessary, the compound (I f) is reacted with alkyl halides such as methyl iodide, ethyl bromide, propyl bromide, isopropyl bromide, allyl chloride, benzyl chloride, and phenethyl chloride, or alkylsulfates such as dimethyl sulfate and diethyl sulfate in the presence of a base such as potassium carbonate and sodium carbonate in an inactive solvent such as acetone, acetonitrile, dimethylformamide, methylene chloride, benzene, and toluene at room temperature or under heating.

## METHOD G

The compound 1 is reacted in the same manner as METHOD A to give the compound 2 as an intermediate, which is subjected to ring closure reaction and dehalogenation to give the compound (II g).

The compound 2 is reacted with iodine in the presence of an alkaline carbonate such as sodium carbonate and potassium carbonate in an inactive solvent such as methylene chloride, chloroform, tetrahydrofuran and benzene under ice-cooling or at room temperature. Then the obtained compound is reacted with tributyltin iodide in the presence of AIBN (2,2'-azobisisobutyronitrile) in benzene under heating to prepare the compound (II g). The compound (II g) is refluxed in the presence of potassium hydroxide or sodium hydroxide in n-butanol, and subjected to hydrolysis and treated with an acid such as hydrochloric acid or sulfuric acid to decarboxylation. The product may be further treated to $R^1$ introduction according to method A (ii).

## METHOD H

The compound (II h) is prepared from the compound 1 through 5 steps then subjected to desulfurization to give the compound ( I h).

The compound (II h) is reacted with Raney® nickel in alcohol under heating to give the compound (I h).

The compound or the present invention can be administered orally or parenterally. For example, the compound of the present invention may be orally administered in the form of tablets, powders, capsules, and granules, aqueous or oily suspension, or liquid form such as syrup or elixir, and parenterally in the from of injection of aqueous or oily suspension.

These preparations can he prepared in a conventional manner by using e.g. excipients, binders, lubricants, aqueous or oily solubilizers, emulsifier and suspending agents. And further preservatives and stabilizers can he used.

The dosages may he varied depending upon the administration route and age, weight, condition, and the kind of disease of the patients, usually 5-1000 mg/day, preferably 20-200 mg/day through oral route, and 1-500 mg/day, preferably 5-50 mg/day through parenteral route in a single or divided doses.

The present invention is illustrated by the following examples and reference examples,

The abbreviations used in examples and reference examples have the following meanings.

Me : methyl, Et : ethyl, Pr : propyl,

iPr : isopropyl, Ph : phenyl, Bz : benzyl

DMF : dimethylformamide, THF : tetrahydrofuran

## Example 1

Spiro(dibenzosuberane-5,2'-pyrrolidine) (I a-1)

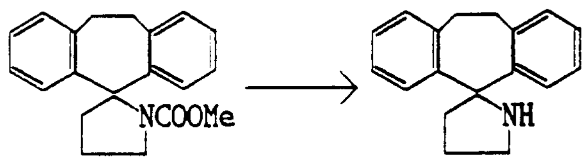

( II a-1) ( I a-1)

A mixture of 5.23 g of spiro[dibenzosuberane-5,2'-(1-methoxycarbonylpyrrolidine)] (IIa-1) and 5.55 g of 86% potassium hydroxide in 78 ml of n-butanol is stirred at 160 °C for 63 hours. After removal of n-butanol under reduced pressure, the residue is diluted with water, acidified with conq. HCl and washed with ether. The ether layer is washed with water for 5 times, and the combined aqueous layer is ride basic with 2N-NaOH and extracted with methylene chloride. The organic layer is washed with water and dried over $MgSO_4$ and evaporated. The residue is subjected to column chromotography on 20 g of silica gel eluting with a mixture of 20% acetonitrile-methylene chloride. The crude crystals 2.07 g are recrystallized from methylene chloride-iPrOH to give 1.95 g (Yield : 46%) of the compound (Ia-1) as white crystals. mp.87-88 °C

## Example 2

Spiro[dibenzosuberane-5,2'-(1-methylpyrrolidine)] (I a-2)

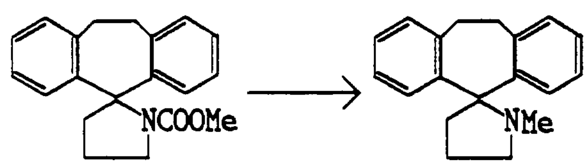

( II a-1) ( I a-2)

A solution of 400 mg of the compound (IIa-1) in 5 ml of THF is added dropwise to a suspension of 99 mg of lithium alminium hydride ( $LiAlH_4$) in 5 ml of THF through 3 minutes, and the mixture is refluxed under beating for 35 minutes on an oil bath. The excess $LiAlH_4$ is decomposed by 2N-NaOH, and the insoluble matter is filtered off. After removal of the solvent under reduced pressure, the residue is subjected to column chromatography of 3 g on silica gel eluting with a mixture of 20 % acetonitrile-methylene chloride. The colorless oily eluate (280 mg) is dissolved in methanol, and the mixture is crystallized by the addition of 115 $\mu\ell$ of 60% perchloric acid. Recrystallization from methanol gives 341 mg (Yield: 72%) of perchlorate of the compound (I a-2) as white crystals. mp. 199-201 °C

Example 3

Spiro[dibenzosuberane-5,2'-(1-ethylpyrrolidine)] (I a-3)

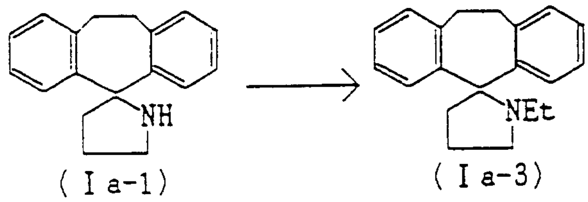

(I a-1) (I a-3)

A mixture of 374 mg of the compound (I a-1) 0.39 ml of diethyl sulfate, and 207 mg of potassium carbonate in 5 ml of acetonitrile is refluxed for 5 hours. To the mixture are added 5 ml of acetonitrile and 0.84 ml of triethylamine, and the reaction mixture is refluxed for additional 30 minutes. After removal of the solvent, to the residue is added water and extracted with ether. The organic layer is washed with water, dried over $MgSO_4$ and concentrated. Then the residue is purified by 5 g of silica gel eluting with a mixture of 10 % acetonitrile and methylene chloride. The resulting 349 mg of colorless oil is crystallized by adding 156 μℓ of 60 % perchloric acid in iso-propanol, and the resulting crystals are recrystallized from methanol-isopropanol to give 510 mg (Yield : 90%) of the perchlorate of the compound (I a-3) as white crystals. mp. 192-194 °C

Examples 4,5

The reactions are performed in the same manner as Example 3 to give the compound (I a-4) and (I a-5). The reaction conditions and products are shown in Table 1.

Example 6

(I a-1)

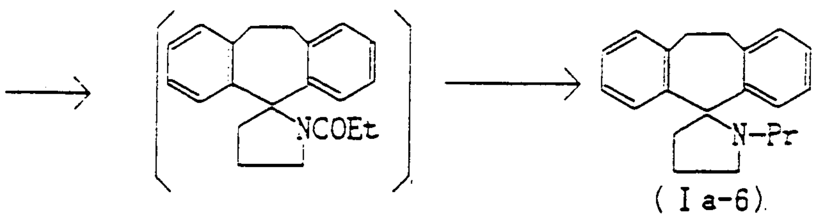

(I a-6)

A mixture of 374 mg of the compound (I a-1), 0.25 ml of triethylamine, and 10 ml of methylene chloride is cooled with ice-water. To a mixture is added 0.14 ml of propionyl chloride, and the mixture is stirred for 1.5 hours. After removal of methylene chloride, the residue is acidified with hydrochloric acid, and extracted with ether. The organic layer is washed with water and dried over $MgSO_4$. After removal of ether, the residue is subjected to column chromatography of 2.5 g on silica gel eluting with a mixture of 20 % acetonitrile and methylene chloride to give 361 mg of spiro(dibenzosuberane-5,2'-(1-propionylpyrrolidine)]. A solution of the resulting product (360 mg) in 5 ml of THF is added to a solution of 56 mg of $LiAlH_4$ in 1 ml of THF. And the reaction mixture is refluxed on an oil bath under heating for 45 minutes. The excess $LiAlH_4$ is dissolved with 2N-NaOH, and the insoluble matter is filtered off. After removal of the solvent, the residue is purified by 5 g of silica gel eluting with methylene chloride. The resulting white oily substance (214 mg) is crystallized out of 80 μℓ of 60 % perchloric acid, and the resulting crystals are recrystallized from methanol to give 258 mg (Yield : 56 %) of the compound (I a-6) as white crystals. mp. 200-202 °C

Example 7

Spiro[dibenzosuberane-5,3'-(1-benzylpyrrolidine)] (I b-1)

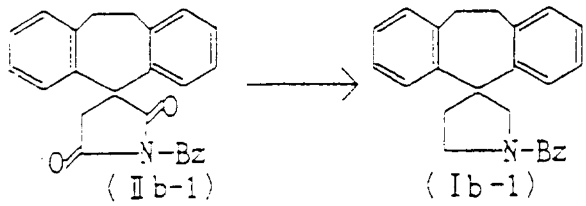

A solution of 2.53 g of the compound (II b-1) in 25 ml of THF is added dropwise to a suspension of 654 mg of $LiAlH_4$ in 15 ml of THF under cooling for 10 minutes and the mixture is refluxed for 1 hour. After decompostion of excess $LiAlH_4$ with 2N-NaOH, the insoluble matter is filtered off. The residue is purified by 39 g of silica gel eluting with toluene and then a mixture 20 % ethyl acetate and toluene. The ethyl acetate-toluene eluate gives 2.26 g of a crystalline product after evaporation. Recrystallization from methylene chloride-propanol gives 2.07 g (Yield : 86%) of the compound (I b-1) as white crystals.
mp. 119- 121 °C

Example 8

Spiro[dibenzosuberane-5,3'-pyrrolidine] (I b-2)

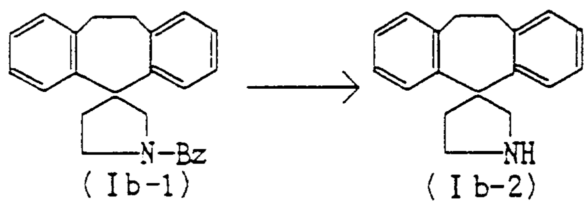

A solution of 1.73 g of the compound (I b-1) in 20 ml of ethylene dichloride is cooled in ice-methanol bath (-15~-16 °C), and a solution of 0.59 ml of α-chloroethyl chloroformate in 6 ml of ethylene dichloride is added dropwise thereto for 5 minutes. The reaction mixture is refluxed on an oil bath under heating for 1 hour, and cooled with ice-water. To the solution is added 13 ml of methanol, and the mixture is heated on an oil bath at 40-42 °C for 2 hours. After removal of methanol under reduced pressure, the crude crystals are recrystallized from methanol-iso-propanol to give 1.31 g (Yield : 90 %) of the compound (I b-2) as white crystals. mp. 270-295 °C (sublimated gradually)

Example 9

Spiro[dibenzosuberane-5,3'-(1-methylpyrrolidine)] (I b-3)

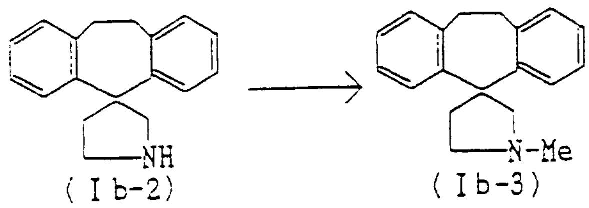

( I b-2) ( I b-3)

A mixture of 286 mg of the compound (I b-2), 75 mg of sodium formate, 0.42 ml of 37% formaline, and 0.42 ml of 90% formic acid is refluxed under heating on an oil bath for 3.5 hours. The reaction mixture is diluted with water, basified with ammonia, and extrated with ether. The resulting crude product (261 mg) is recrystallized from ether-isopropanol to give 175 mg (Yield : 67%) of the compound (I b-3) as white crystals. mp. 81-82 °C

Examples 10-15

The reactions are performed in the same manner as Example 7 to give the compound (I b). The reaction conditions and products are shown in Table 2.

Example 16

Spiro[dibenzosuberane-5,2'-azetidine) (I c-1)

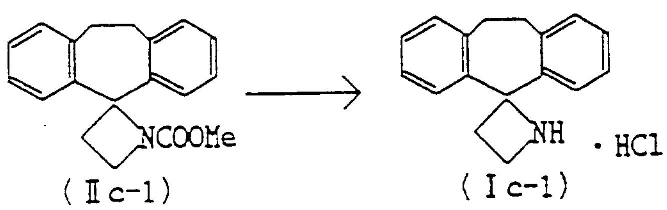

( II c-1) ( I c-1)

A mixture of 2.44 g of the compound (II c-1), 2.71 g of 86% potassium hydroxide, and 24 ml of n-butanol is refluxed under heating on an oil bath for 47 hours. Then the reaction mixture is treated in the same manner as Example 1 to give 1.43 g (Yield : 63%) of hydrochloride of the compound (I c-1). mp. 217-218 °C

Example 17

Spiro[dibenzosuberane-5,2'-(1-methylazetidine)] (I c-2)

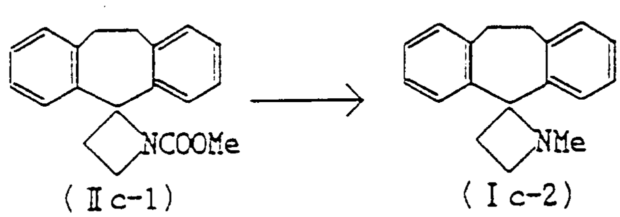

( II c-1) ( I c-2)

According to the method shown in Example 2, 300 mg of the compound (II c-1) is treated with 77 mg of $LiAlH_4$ in 2 ml of THF to give 194 mg (Yield : 71%) of the compound (I c-2). mp. 108-109 °C

Examples 18, 19

Spiro[dibenzosuberane-5,2'-(1-allylazetidine)] (I c-3)

Spiro[dibenzosuberane-5,2'-(1-benzylazetidine)] (I c-4)

The reactions are performed in the same manner as Example 3 to give the compounds (I c-3) and (I c-4). The reaction conditions and products are shown in Table 1.

Example 20

Spiro[dibenzosuberane-5,3'-azetidine)] (I d-1)

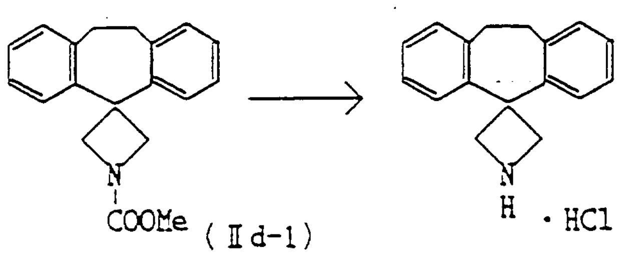

(II d-1) (I d-1)

A mixture of 2.65 g of the compound (II d-1), 2.94 g of 86% potassium hydroxide, and 27 ml of n-butanol is refluxed under heating on an oil bath for 15.5 hours. Then the reaction mixture is treated in the same manner as Example 1 to give 2.03 g.(Yield : 83%) of hydrochloride of the compound (I d-1) as white crystals. mp.250-252 °C

Example 21

Spiro[dibenzosuberane-5,3'-(1-methylazetidine)] (I d-2)

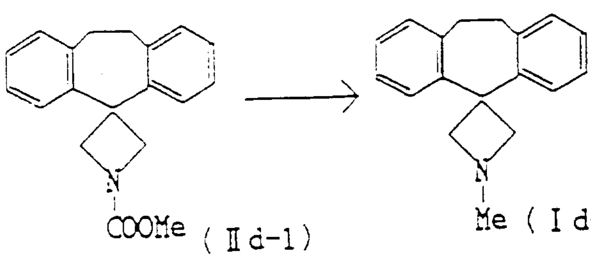

(II d-1) (I d-2)

According to Example 2, 339 mg of the compound (II d-1) is treated with 88 mg of $LiAlH_4$ in 2 ml of THF to give 300 mg (Yield : 74%) of the compound (I d-2). mp. 179-180 °C

Examples 22, 23

Spiro[dibenzosuberane-5,3'-(1-n-propylazetidine)] (I d-3)

Spiro[dibenzosuberane-5,3'-(1-phenethylazetidine) (I d-4)

The reactions are performed in the same manner as Example 3 to give the compounds (I d-3) and (I d-4). The reaction conditions and products are shown in Table 1.

Example 24

Spiro[dibenzosuberane-5,2'-piperidine) (I e-1)

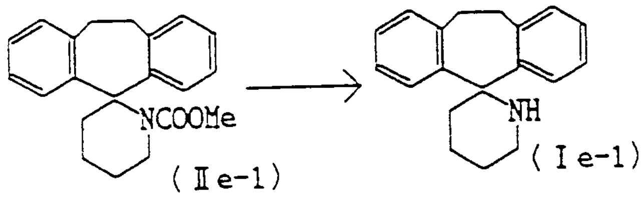

(II e-1) (I e-1)

A mixture of 4.93 g of the compound (II e-1), 5.0 g of 86 % of potassium hydroxide, and 50 ml of butanol is refluxed under heating on an oil bath at 160-162 °C for 68 hours. Then the reaction mixture is treated in the same manner as Example 1 to give 2.46 g (Yield : 61%) of the compound (I e-1) as white crystals. mp. 107-108 °C

Example 25

Spiro[dibenzosuberane-5,2'-(1-methylpiperidine)] (I e-2)

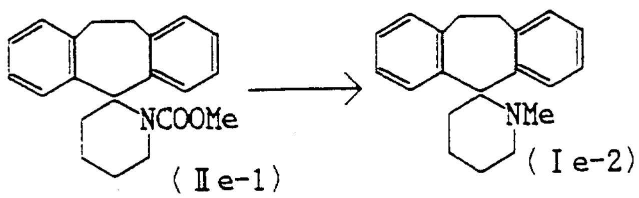

(II e-1) (I e-2)

As a starting material, the compound (II e-1) 400mg is reacted in the same manner as Example 2 in a suspension of 94 mg of $LiAlH_4$ in 5 ml of THF to give 404 mg (Yield : 86%) of the compound (I e-2). mp. 182-184 °C

Examples 26, 27

Spiro[dibenzosuberane-5,2'-(1-allylpiperidine)] (I e-3)

Spiro[dibenzosuberane-5,2'-(1-benzylpiperidine] (I e-4)

The reactions are performed in the same manner as Example 3 to give the compounds ( I e-3) and (I e-4). The reaction conditions and products are shown in Table 1.

Reference Example 10

Spiro[dibenzosuberane-5,3'-piperidine) (I f-1)

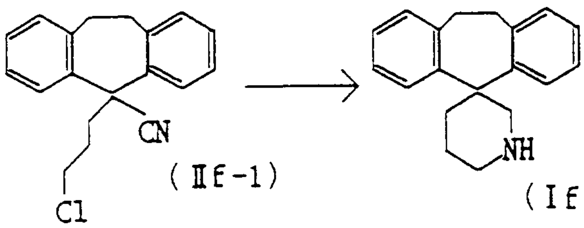

( IIf-1)

( If-1)

A solution of 5.9 g of the compound (II f-1) in 40 ml of ether is added dropwise to a mixture of 759 mg of $LiAlH_4$ and 2.67 g of aluminium chloride in 60 ml of ether under cooling, and reaction mixture is stirred at room temperature for 1 hour. After decomposition of excess $LiAlH_4$ with 60ml of 2N-NaOH the reaction solution is washed with water and extracted with c.HCl. The aqueous layer is basified with 2N-NaOH and extracted with methylene chloride. The organic layer is washed with water, and dried over $MgSO_4$ and evaporated under reduced pressure. The resulting white powder 4.68 g is dissolved in 47 ml of dimethylformamide, and stirred with 2.59 g of potassium carbonate at room temperature for 15 hours. The reaction mixture is diluted with water and extracted with toluene. The organic layer is washed with water, dried over $MgSO_4$ and evaporated. The residue is purified by 29 g of alumina and eluted with a mixture of 20 % acetonitrile-methylene chloride. The eluate 4.07 g is dissolved in isopropanol and treated with 1.25 ml of c.HCl. The resulting crystals are recrystallized from methanol/isopropanol to give 3.01 g (Yield : 50%) of the compound (I f-1) as white crystals. mp. 257-263 °C

Example 29

Spiro[dibenzosuberane-5,3'-(1-methylpiperidine)] (I f-2)

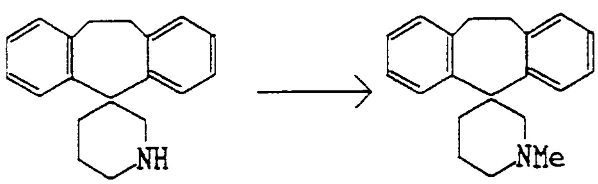

( If-1)

( If-2)

A mixture of 450 mg of the compound (I f-1) obtained in Reference Example 10 112 mg of sodium formate, 0.68 ml of 37 % of formaline, and 0.64 ml of 90% formate is refluxed under heating on an oil bath for 2.5 hours. To the mixture is added ice-water, and the solution is basified with ammonium and extracted with methylene chloride. The methylene chloride layer is washed with water, dried over $MgSO_4$, purified by a column chromatography of 6 g of silica gel eluting with a mixture of 20 % acetonitrile-methylene chloride to give 400 mg of crude product. It is recrystallized from ether-isopropanol to give 377 mg (Yield : 91 %) of the compound (I f-2) as white crystals. mp. 145-146 °C

Examples 30, 31

Spiro[dibenzosuberane-5,3'-(1-n-propylpiperidine)] (I f-3)

Spiro[dibenzosuberane-5,3'-(1-phenethylpiperidine)] (I f-4)

The reactions are performed in the same manner as Example 3 to give the compounds (I f-3) and (I f-4). The reaction conditions and products are shown in Table 1.

Example 32

Spiro[dibenzosuberane-5,2'-(4-methylazetidine)] (I g-1)

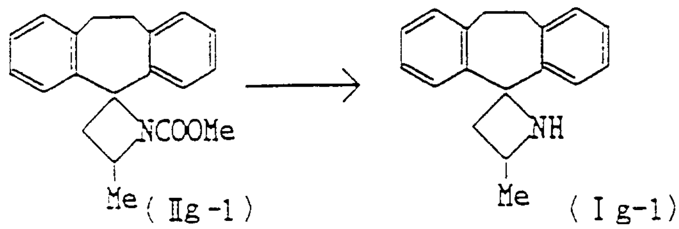

( IIg -1 ) ( I g-1 )

A mixture of 1.50 g of the compound (II g-1), 1.37 g of potassium hydroxide, and 15 ml of n-butanol is refluxed under heating for 6 days. The reaction mixture is treated in the same manner as Example 1 to give 634 mg (Yield : 49%) of the compound (I g-1) as white crystals. mp. 116-118 °C

Example 33

Spiro[dibenzosuberane-5,2'-(5-methylpyrrolidine)] (I h-1)

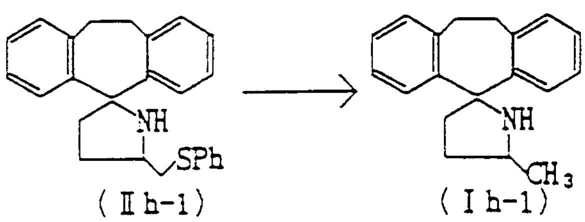

( II h-1 ) ( I h-1 )

A mixture of 2.78 g of the compound (II h-1), 15 ml of Raney® nickel/ethanol, and 50 ml of ethanol is refluxed on an oil bath under heating for 2 hours. Then Raney® nickel is filtered off and the solvent is removed. The residue is purified by 25 g of silica gel eluting with a mixture of 5% acetonitrile-methylene chloride to give 1.19 g of the eluate. It is crystallized by 0.52 ml of 60 % perchloric acid and recrystallized from methanol/isopropanol to give 1.58 g (Yield : 59%) of the compound ( I h-1) as white crystals. mp. 211-212 °C

Example 34

Spiro[3-methylbenzosuberane-5,2'-pyrrolidine) (I a-7)

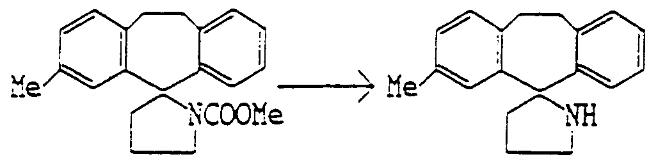

( II a-2)    ( I a-7)

A mixture of 7.40 g of spiro[3-methyldibenzosuberane-5,2'-(1-methoxycarbonylpyrrolidine)] (II a-2) and 7.33 g of 86 % of potassium hydroxide in 74 ml of n-butanol is refluxed at 160 °C for 146 hours. After removal of n-butanol under reduced pressure, the residue is diluted with water, and acidified with c.HCl and washed with ether. The ether layer is washed with water for 5 times. The combined aqueous layer is basified with 2N-NaOH and extracted with methylene chloride. The organic layer is washed with water, dried over $MgSO_4$ and evaporated. The residue is chromatographed on silica gel eluting with 20% acetonitrile-methylene chloride to give 4.16 g (Yield : 70%) of the compound (I a-7). The obtained oily substance (320 mg) is reacted with 60 % $HClO_4$ in i-PrOH to give 308 mg of perchlorate as white crystals.
mp. 190-191 °C

Example 35

Spiro[3-methyldibenzosuberane-5,2'-(1-methylpyrrolidine) (I a-8)

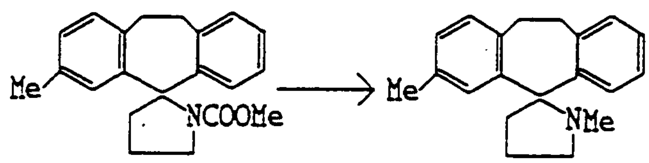

( II a-2)    ( I a-8)

A solution of 986 mg of the compound (II a-2) in 12 ml of THF is added dropwise to a suspension of 227 mg of $LiAlH_4$ in 12 ml of THF for 7 minutes, and the mixture is refluxed under heating on an oil bath for 11 hours. After decomposition of excess $LiAlH_4$ with 2N-NaOH, the insoluble substance is filtered off. After removal of the solvent under reduced pressure, the residue is subjected to column chromatography over silica gel eluting with 20 % acetonitrile-methylene chloride. The obtained colorless oily substance (326 mg) is dissolved in methanol and mixed with 140 μℓ of 60 % perchloric acid to crystallization. It is recrystallized from methanol-isopropanol to give 336 mg (Yield : 30 %) of the compound (I a-8) as white crystals. mp. 198.0-199.0 °C

Example 36

Spiro[3-methyldibenzosuberane-5,2'-(1-n-propylpyrrolidine)] (I a -9)

( I a-7)
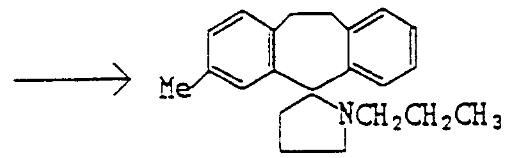
( I a-9)

(1) A mixture of 509 mg of the compound (I a-7), 0.40 ml of triethylamine, and 15 ml of methylene chloride is cooled with ice-water. To the mixture is added 0.22 ml of propionyl chloride, and the mixture is stirred for 1.5 hours. After removal of methylene chloride, the residue is acidified with HCl and extracted with ether. The organic layer is washed with water and dried to remove ether. The residue is subjected to column chromatography of silica gel eluting with 20 % ethyl acetate-toluene to give 684 mg (Yield : 100%) of spiro[3-methyldibenzosuberane-5,2'-(1-propionylpyrrolidine)] as white oily substance.
(2) A solution of 684 mg of the obtained compound in 9 ml of THF is added dropwise to a suspension of 88 mg of $LiAlH_4$ in 3 ml of THF, and the mixture is refluxed under heating on an oil bath for 1 hour. After decomposition of excess $LiAlH_4$ with 2N-NaOH, the insoluble matter is filtered off and concentrated to remove the solvent. The residue is subjected to column chromatography of silica gel eluting with 10 % ethyl acetate-toluene. The white oily substance 465 mg, is crystallized from 182 μℓ of 60 % perchloric acid. The resulting crystals are recrystallized from methanol-ether to give 500 mg (Yield : 64 %) of the compound (I a-9). mp.186-187 °C

Examples 37-39

The compound (I a-7) is reacted in the same manner as Example 35 to give the compounds (I a-10), (I a-11) and (I a-12). The reaction conditions and products are shown in Table 3.

Example 40

Spiro(3-chlorodibenzosuberane-5,2'-pyrrolidine) (I a-13)

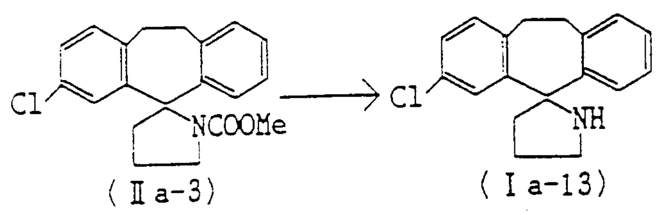
( II a-3) ( I a-13)

A mixture of 9.53 g of spiro[3-chlorodibenzosuberane-5,2'-(1-methoxycarbonylpyrrolidine) (II a-3), and 7.67 g of 86 % potassium hydroxide in 95 ml of n-butanol is refluxed at 160 °C on an oil bath under heating for 240 hours. After removal of n-butanol under reduced pressure, to the residue is added water. The solution is acidified with c.HCl and extracted with ether. The ether layer is washed with water for 5 times, and the solution is basified with 2N-NaOH and extracted with ethyl acetate. The organic layer is washed with water, dried over $MgSO_4$. The resulting crude crystals are recrystallized from ether to give 4.54 g (Yield : 59%) of the compound (I a-13) as white crystals. mp. 102-104 °C

Example 41

Spiro[3-chlorodibenzosuberane-5,2'-(1-ethylpyrrolidine) (I a-14)

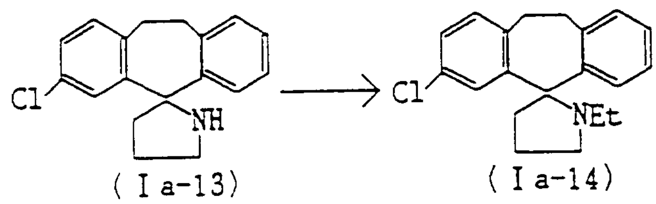

( I a-13) ( I a-14)

A mixture of 410 mg of the compound (I a-13), 0.38 ml of diethyl sulfate, 415 mg of potassium carbonate in 7 ml of acetonitrile is refluxed on an oil bath under heating for 16 hours. To the reaction mixture are added 5 ml of acetonitrile and 1.5 ml of triethylamine, and the mixture is refluxed under heating further one 1 hour. After removal of the solvent, to the residue is added water. The solution is extracted with ethyl acetate. The organic layer is washed with water, dried over $MgSO_4$ and concentrated to remove ethyl acetate. The residue is subjected to column chromatography of silica gel eluting with 10% ethyl acetate-toluene. The obtained colorless oily substance (0.40 g) is crystallized from 0.5 g of 60% perchloric acid in i-PrOH, the resulting crystals are recrystallized from MeOH-iPrOH to give 0.50 g (Yield : 84%) of the compound (I a-14) as white crystals. mp. 205-207 °C

Example 42

Spiro(3-bromodibenzosuberane-5,2'-pyrrolidine)hydrobromate (I a-15)

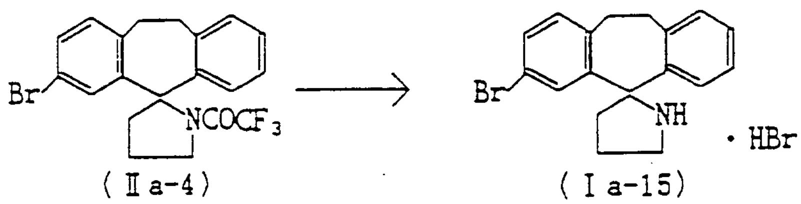

( II a-4) ( I a-15)

A mixture of 7.23 g of the compound (II a-4) and 1.23 g of sodium borohydride in 140 ml of 95% ethanol is stirred on an oil bath at 50-53 °C for 15 hours. After removal of ethanol under reduced pressure, to the residue is added water. The solution is extracted with methylene chloride, washed with water, and dried over $MgSO_4$. The residue is subjected to column chromatography of silica gel eluting with a mixture of 10 % acetonitrile-methylene choride. The eluate (4.88 g) is dissolved in ether and treated with 1.7 ml of hydrobromic acid. The obtained hydrobromate is recrystallized from methanol/2-propanol to give 5.70 g (Yield : 86%) of the compound (I a-15).

| Anal Calcd. (%) for $C_{18}H_{18}NBr \cdot HBr$ | | | | |
|---|---|---|---|---|
| : | C,52.83; | H,4.68; | N,3.42; | Br,39.06 |
| Found : | C,52.67; | H,4.73; | N,3.68; | Br,38.83 |

IR $\nu$ (Nujol) : 2660, 1586, 1484, 1453, 1390, 823, 762, 740 $cm^{-1}$

Example 43

Spiro(2-chlorodibenzosuberane-5,2'-pyrrolidine)sulfate (I a-16)

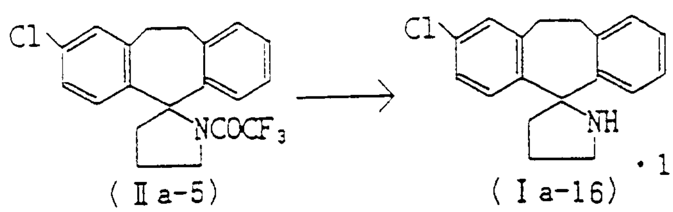

( II a-5) ( I a-16) · $1/2H_2SO_4$ · $1/2H_2O$

A mixture of 3.25 g of the compound (II a-5) and 324 mg of sodium borohydride in 65 ml of 95% ethanol is stirred on an oil bath at 50-53 °C for 48 hours. After removal of ethanol under reduced pressure, to the residue is added water. The solution is extracted with ethylene chloride, washed with water, and dried over $MgSO_4$. The residue is subjected to column chromatography of silica gel eluting with 20% acetonitrile-methylene chloride. The eluate (2.35 g) is dissolved in ether and treated with 400 mg of sulfuric acid. The sulfate is recrystallized from methanol/2-propanol to give 2.12 g (Yield : 72%) of the compound (I a-16) as white crystals. mp. 163-165 °C

| Anal Calcd. (%) for $C_{18}H_{18}NCl$ • $1/2H_2SO_4$ • $1/2H_2O$ | | | | | |
|---|---|---|---|---|---|
| : | C, 63.24; | H,5.90; | N,4.10; | Cl, 10.37; | S,4.69 |
| Found : | C, 63.16; | H,6.01; | N,4.34; | Cl, 10.37; | S,4.59 |

IR ν (Nujol) :
3400, 2600, 2460, 1479, 1459, 1380, 1131, 1095, 1013, 961 $cm^{-1}$

Example 44

Spiro[dibenzosuberane-5,2'-(3-methylpyrrolidine)]perchlorate (I a-17)

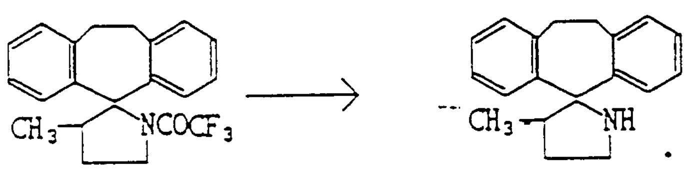

( II a-6) ( I a-17) · $HClO_4$

A mixture of 1.18 g of the compound (II a-6) and 248 mg of sodium borohydride in 24 ml of 95 % ethanol is stirred on an oil bath at 50-53 °C for 47 hours. After removal of ethanol under reduced pressure, to the residue is added water. The solution is extracted with ethylene chloride, washed with water, and dried over $MgSO_4$. The residue is subjected to column chromatography of silica gel eluting with a mixture of 10% acetonitrile-methylene choride. The eluate (814 mg) is dissolved in ether and treated with 0.34 ml of 60% perchloric acid. The perchlorate is recrystallized from methanol/2-propanol to give 1.04 g (Yield : 87%) of the compound (I a-17) as white crystals.
mp. : 278-281 °C (decomposition)

| Anal Calcd. (%) for $C_{19}H_{21}N \cdot HClO_4$ | | | | |
|---|---|---|---|---|
| : | C,62.72; | H,6.10; | N,3.85; | Cl,9.75 |
| Found : | C,62.66; | H,6.06; | N,3.96; | Cl,9.80 |

IR ν (Nujol) :
3070, 1620, 1462, 1395, 1386, 1134, 1053, 758, 747 $cm^{-1}$

Table 1

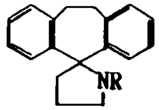
(Ia)
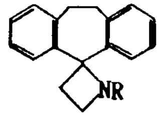
(Ic)
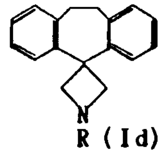
(Id)
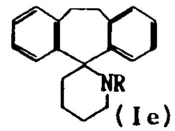
(Ie)
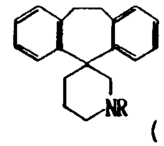
(If)

| Ex. No. | starting material | agent | $K_2CO_3$ | aceto-nitrile | reaction condition | product (compd. No.) mg (Yield) |
|---|---|---|---|---|---|---|
| 4 | I a − 1 249 mg | $PhCH_2Br$ 0.24 ml | 138 mg | 5 ml | 80 minutes reflux | I a-4 (R: $-CH_2Ph$) 270mg (61%) |
| 5 | I a − 1 249 mg | $CH_2CH=CH_2Br$ 0.17 ml | 138 mg | 10 ml | 6 hours reflux | I a-5 (R: $-CH_2CH=CH_2$) 257mg (69%) |
| 18 | I c − 1 306 mg | $CH_2CH=CH_2Br$ 0.34 ml | 180 mg | 5 ml | 16 hours reflux | I c-3 (R: $-CH_2CH=CH_2$) 344mg (80%) |
| 19 | I c − 1 282 mg | $PhCH_2Br$ 0.29 ml | 166 mg | 5 ml | 6 hours reflux | I c-4 (R: $-CH_2Ph$) 355 mg (78 %) |
| 22 | I d − 1 306 mg | $n-C_3H_7Br$ 0.15 ml | 359 mg | 5 ml | 1 hours reflux | I d-3 (R: $n-C_3H_7$) 413 mg (84 %) |
| 23 | I d − 1 306 mg | $CH_2CH_2PhBr$ 0.23 ml | 359 mg | 5 ml | 2 hours reflux | I d-4 (R: $-CH_2CH_2Ph$) 409 mg (72 %) |
| 26 | I e − 1 300 mg | $CH_2CH=CH_2Br$ 0.20 ml | 156 mg | 5 ml | 4 hours reflux | I e-3 (R: $-CH_2CH=CH_2$) 389mg (85%) |
| 27 | I e − 1 263 mg | $PhCH_2Br$ 0.24 ml | 138 mg | 5 ml | 4 hours reflux | I e-4 (R: $-CH_2Ph$) 301 mg (85 %) |
| 30 | I f − 1 395 mg | $n-C_3H_7Br$ 0.18 ml | 207 mg | 7 ml | 2 hours reflux | I f-3 (R: $n-C_3H_7$) 397 mg (77 %) |
| 31 | I f − 1 394 mg | $PhCH_2CH_2Br$ 0.27 ml | 207 mg | 7 ml | 2 hours reflux | I f-4 (R: $-CH_2CH_2Ph$) 469mg (85%) |

Table 2

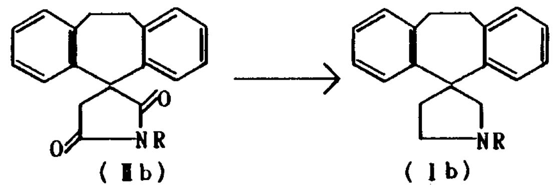

( IIb ) ( Ib )

| Ex. No. | starting material | reagent | $LiAlH_4$ | THF | reaction condition | product (compd. No.) mg (Yield) |
|---|---|---|---|---|---|---|
| 10 | IIb - 2 393 mg | (R=-Et) | 122 mg | 7 ml | 1 hour reflux | Ib-4 263 mg (74 %) |
| 11 | IIb - 3 438 mg | (R=-n-$C_3H_7$) | 130 mg | 7 ml | 1 hour reflux | Ib-5 319 mg (80 %) |
| 12 | IIb - 4 417 mg | (R=-i-$C_3H_7$) | 124 mg | 7 ml | 1 hour reflux | Ib-6 289 mg (76 %) |
| 13 | IIb - 5 445 mg | (R=-$CH_2$=$CHCH_2$) | 143 mg | 7 ml | 1 hour reflux | Ib-7 351 mg (85 %) |
| 14 | IIb - 6 422 mg | (R=-$CH_2CH_2Ph$) | 106 mg | 7 ml | 1 hour reflux | Ib-8 381 mg (76 %) |
| 15 | IIb - 7 383 mg | (R=-$CH_2$- 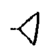 ) | 110 mg | 7 ml | 1 hour reflux | Ib-9 319 mg (91 %) |

Table 3

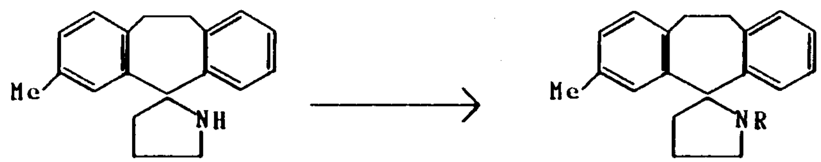

( I a-7)

| Ex. No. | starting material | reagent | $K_2CO_3$ | aceto-nitrile | reaction condition | Product (Compd. No.) mg (Yield) |
|---|---|---|---|---|---|---|
| 3 7 | I a − 7 398 mg | $(C_2H_5O)_2SO_2$ 0.40 ml | 209 mg | 7 ml | 8 hours reflux | I a-10 (R: $-C_2H_5$) 506mg (85 %) |
| 3 8 | I a − 7 320 mg | $PhCH_2Br$ 0.29 ml | 168 mg | 6 ml | 3 hours reflux | I a-11 (R: $-CH_2Ph$) 284 mg (52 %) |
| 3 9 | I a − 7 375 mg | $CH_2=CHCH_2Br$ 0.246 ml | 197 mg | 14 ml | 11 hours reflux | I a-12 (R: $-CH_2CH=CH_2$) 459 mg (80 %) |

Table 4 (No. 1)

| Compd. No. | m.p. (°C) (solvent)*1 | Anal. Calcd. (%) Found (%) | IR ($cm^{-1}$) | $^1H$-NMR ($CDCl_3$) (δ) |
|---|---|---|---|---|
| I a-1 | 87.0-88.0 ($CH_2Cl_2$-iPrOH) | $C_{18}H_{19}N$<br>C, 86.75 (86.70)<br>H, 7.77 (7.68)<br>N, 5.73 (5.62) | ($CHCl_3$)<br>3370, 1601, 1479<br>1444 | 7.53-7.61 (2H, m); 7.06-7.14 (6H, m); 3.43-3.60 (2H, m); 3.13-3.30 (2H, m); 2.54-2.61 (2H, m); 2.27 (1H, brs); 1.75-1.90 (2H, m) |
| I a-2 | 199.0-201.0 (MeOH) | $C_{19}H_{21}N \cdot HClO_4$<br>C, 62.62 (62.72)<br>H, 6.13 (6.10)<br>N, 4.05 (3.85)<br>Cl, 9.58 (9.75) | (Nujol)<br>3200, 1462, 1451<br>1120, 1099, 1061<br>751 | 7.32-7.36 (2H, m); 7.07-7.25 (6H, m); 3.24 (2H, t); 3.04-3.36 (4H, m); 2.45 (2H, t); 2.34 (3H, s); 1.79 (2H, quint) |
| I a-3 | 192.0-194.0 (MeOH-iPrOH) | $C_{20}H_{23}N \cdot HClO_4$<br>C, 63.46 (63.57)<br>H, 6.41 (6.40)<br>N, 3.94 (3.71)<br>Cl, 9.38 (9.38) | (Nujol)<br>3160, 1468, 1373<br>1103, 1069, 780<br>754 | 7.39-7.44 (2H, m); 7.05-7.16 (6H, m); 2.99-3.14, 3.33-3.49 (4H, m) 3.12 (2H, t); 2.45 (2H, t); 2.44 (2H, q); 1.81 (2H, quint); 1.12 (3H, t) |
| I a-4 | 176.0-177.0 (MeOH-iPrOH) | $C_{25}H_{25}N \cdot HClO_4$<br>C, 68.28 (68.25)<br>H, 6.00 (5.96)<br>N, 3.40 (3.18)<br>Cl, 7.96 (8.06) | (Nujol)<br>3170, 1457, 1372<br>1119, 1099, 1066<br>772, 748 | 7.08-7.43 (13H, m); 3.01, 3.82 (4H, m); 3.55 (2H, s); 2.85 (2H, t); 2.70 (2H, t); 1.87 (2H, m) |
| I a-5 | 198.0-199.0 (MeOH) | $C_{21}H_{23}N \cdot HClO_4$<br>C, 64.47 (64.69)<br>H, 6.29 (6.20)<br>N, 3.79 (3.59)<br>Cl, 9.11 (9.09) | (Nujol)<br>3145, 1457, 1374<br>1111, 1060, 753 | 7.34-7.42 (2H, m); 7.08-7.17 (6H, m); 5.86-6.07 (1H, m); 5.03-5.21 (2H, m); 2.94-3.11, 3.45-3.61 (4H, m); 2.30-3.04 (4H, m); 2.52 (2H, t); 1.82 (2H, quint) |

Table 4 (No. 2)

| Compd. No. | m.p. (°C) (solvent*1) | Anal. Calcd. (%) Found (%) | IR (cm⁻¹) | ¹H-NMR ($CDCl_3$) (δ) |
|---|---|---|---|---|
| I a-6 | 200.0-202.0 (MeOH) | $C_{21}H_{26}N \cdot HClO_4$<br>C, 64.28 (64.36)<br>H, 6.69 (6.69)<br>N, 3.82 (3.57)<br>Cl, 8.91 (9.05) | (Nujol)<br>3165, 1470, 1378<br>1366, 1119, 1106<br>1065, 755 | 7.41-7.45 (2H, m); 7.05-7.16 (6H, m); 2.98-3.14, 3.31-3.46 (4H, m) 3.15 (2H, t); 2.43 (2H, t); 2.30 (2H, m); 1.80 (2H, quint); 1.60 (2H, m); 0.80 (3H, t) |
| I a-7 | 190.0-191.0 (iPrOH-$Et_2O$) | $C_{19}H_{21}N \cdot HCl$<br>C, 62.49 (62.72)<br>H, 6.10 (6.09)<br>N, 3.86 (3.85)<br>Cl, 9.62 (9.74) | (Nujol)<br>3076, 1622, 1611<br>1598, 1503, 1494<br>1455, 1410 | 6.85-7.60 (7H, m); 3.40-3.65 (2H, m); 3.24 (2H, t); 3.03-3.30 (4H, m); 2.45-2.70 (2H, m); 2.28 (3H, s); 2.05 (1H, brs); 1.83 (2H, q) |
| I a-8 | 198.0-199.0 (MeOH-iPrOH) | $C_{20}H_{23}N \cdot HClO_4$<br>C, 63.44 (63.57)<br>H, 6.42 (6.40)<br>N, 3.74 (3.71)<br>Cl, 9.26 (9.38) | (Nujol)<br>3160, 3037, 1612<br>1503, 1480, 1466<br>1453, 1434, 1428 | 6.78-7.40 (7H, m); 2.95-3.42 (6H, m); 2.46 (2H, t); 2.33 (3H, s); 2.31 (3H, s); 1.81 (2H, q) |
| I a-9 | 177.0-178.0 (MeOH-$Et_2O$) | $C_{21}H_{26}N \cdot HClO_4$<br>C, 64.23 (64.36)<br>H, 6.59 (6.69)<br>N, 3.64 (3.57)<br>Cl, 8.99 (9.05) | (Nujol)<br>3145, 3065, 1612<br>1503, 1484, 1468<br>1452, 1428, 1417 | 6.85-7.45 (7H, m); 3.30-3.50 (2H, m); 2.87-3.20 (4H, m); 2.33-2.53 (4H, m); 2.30 (3H, s); 1.81 (2H, q); 1.11 (3H, t) |
| I a-10 | 186.0-187.0 (MeOH-$Et_2O$) | $C_{22}H_{27}N \cdot HClO_4$<br>C, 64.96 (65.10)<br>H, 6.84 (6.95)<br>N, 3.55 (3.45)<br>Cl, 8.61 (8.73) | (Nujol)<br>3150, 1613, 1501<br>1482, 1471, 1458<br>1428, 1417 | 6.80-7.50 (7H, m); 3.20-3.50 (2H, m); 2.80-3.20 (4H, m); 2.30-2.55 (4H, m); 2.30 (3H, s); 1.80 (2H, quint); 1.58 (2H, quint); 0.80 (3H, t) |

Table 4 (No. 3)

| Compd. No. | m.p. (°C) (solvent) | Anal. Calcd. (%) Found (%) | IR ($cm^{-1}$) | $^{1}H$-NMR ($CDCl_3$) ($\delta$) |
|---|---|---|---|---|
| I a-11 | 158.0-160.0 (iPrOH-$Et_2O$) | $C_{26}H_{27}N \cdot HClO_4$<br>C, 68.69 (68.79)<br>H, 6.26 (6.22)<br>N, 3.11 (3.09)<br>Cl, 7.75 (7.81) | (Nujol)<br>3150, 3045, 1610<br>1501, 1481, 1459<br>1427, 1410 | 6.85-7.45 (7H, m); 3.61-3.90 (2H, m); 3.51, 3.60 (2H, ABq); 2.77-3.08 (4H, m); 2.69 (2H, t); 2.27 (3H, s); 1.86 (2H, quint) |
| I a-12 | 168.0-170.0 (MeOH-$Et_2O$) | $C_{22}H_{26}N \cdot HClO_4$<br>C, 65.19 (65.42)<br>H, 6.47 (6.49)<br>N, 3.52 (3.47)<br>Cl, 8.65 (8.78) | (Nujol)<br>3135, 3025, 1646<br>1617, 1506, 1485<br>1457, 1433, 1427 | 6.85-7.40 (7H, m); 5.84-6.08 (1H, m); 5.00-5.24 (2H, m); 3.42-3.64 (2H, m); 2.85-3.13 (6H, m); 2.53 (2H, t); 2.29 (3H, s); 1.82 (2H, quint) |
| I a-13 | 102.0-104.0 (iPrOH-$Et_2O$) | $C_{18}H_{18}NCl$<br>C, 76.18 (76.18)<br>H, 6.44 (6.39)<br>N, 5.00 (4.94)<br>Cl, 12.52 (12.49) | ($CHCl_3$)<br>3690, 3380, 1592<br>1565, 1476, 1431<br>1398 | 6.96-7.66 (7H, m); 3.36-3.63 (2H, m); 3.04-3.24 (4H, m); 2.43-2.64 (2H, m); 2.19 (1H, brs); 1.82 (2H, quint) |
| I a-14 | 205.0-207.0 (iPrOH) | $C_{20}H_{22}NCl \cdot HClO_4$<br>C, 58.18 (58.26)<br>H, 5.66 (5.62)<br>N, 3.44 (3.40) | (Nujol)<br>3150, 1594, 1560 | 6.95-7.48 (7H, m); 2.93-3.40 (6H, m); 2.30-2.54 (4H, m); 1.81 (2H, quint); 1.13 (3H, t) |
| I b-1 | 119.0-121.0 ($CH_2Cl_2$-iPrOH) | $C_{25}H_{25}N$<br>C, 88.58 (88.45)<br>H, 7.39 (7.42)<br>N, 4.17 (4.13) | ($CHCl_3$)<br>1603, 1495, 1484<br>1453 | 7.05-7.46 (13H, m); 3.74 (2H, s); 3.42-3.49 (2H, brs); 2.98-3.12, 3.49-3.74 (4H, m); 2.70-2.86 (4H, m) |

Table 4 (No. 4)

| Compd. No. | m.p. (°C) (solvent) | Anal. Calcd. (%) Found (%) | IR (cm$^{-1}$) | $^1$H-NMR ($CDCl_3$) (δ) |
|---|---|---|---|---|
| I b-2 | 270.0 (sublimation) (MeOHiPrOH) | $C_{18}H_{19}N \cdot HCl$<br>C, 75.50 (75.64)<br>H, 7.06 (7.05)<br>N, 5.01 (4.90)<br>Cl, 12.71 (12.41) | (Nujol)<br>2710, 2660, 2585<br>2440, 1493, 1470<br>1460, 783, 756 | 9.55 (2H, brs); 7.25-7.31 (2H, m); 7.09-7.17 (6H, m); 4.03 (2H, s) 3.15-3.46 (6H, m); 2.88 (2H, t) |
| I b-3 | 81.0-82.0 ($Et_2O$-iPrOH) | $C_{19}H_{21}N$<br>C, 86.39 (86.64)<br>H, 8.22 (8.04)<br>N, 5.50 (5.32) | ($CHCl_3$)<br>1603, 1485, 1451<br>1358, 1148, 1125<br>1054, 1043 | 7.31-7.36 (2H, m); 7.05-7.11 (6H, m); 3.45 (2H, m); 2.99-3.15, 3.43-3.59 (4H, m); 2.75 (4H, s); 2.47 (3H, s) |
| I b-4 | 58.0-59.0 ($Et_2O$-iPrOH) | $C_{20}H_{23}N$<br>C, 86.55 (86.59)<br>H, 8.37 (8.36)<br>N, 5.28 (5.05) | ($CHCl_3$)<br>1600, 1483, 1448<br>1387, 1175, 1149 | 7.33-7.38 (2H, m); 7.05-7.11 (6H, m); 3.48 (2H, s); 3.00-3.16, 3.42-3.61 (4H, m); 2.69-2.82 (4H, m); 2.60 (2H, q); 1.23 (3H, t) |
| I b-5 | 74.0-75.0 ($Et_2O$-iPrOH) | $C_{21}H_{25}N$<br>C, 86.51 (86.55)<br>H, 8.67 (8.65)<br>N, 5.01 (4.81) | ($CHCl_3$)<br>1603, 1485, 1469<br>1450, 1444, 1390<br>1358 | 7.33-7.38 (2H, m); 7.06-7.11 (6H, m); 3.47 (2H, s); 2.97-3.14, 3.45-3.62 (4H, m); 2.51 (2H, t); 1.64 (2H, m); 0.98 (3H, t) |
| I b-6 | 90.0-91.0 ($Et_2O$-iPrOH) | $C_{21}H_{25}N$<br>C, 86.59 (86.55)<br>H, 8.74 (8.65)<br>N, 4.83 (4.81) | ($CHCl_3$)<br>1485, 1451, 1386<br>1372, 1330, 1183 | 7.35-7.40 (2H, m); 7.05-7.10 (6H, m); 3.54 (2H, s); 3.00-3.18, 3.42-3.58 (4H, m); 2.81, 2.72 (4H, m); 2.52 (1H, m); 1.18 (6H, d) |

Table 4 (No. 5)

| Compd. No. | m.p. (°C) (solvent) | Anal. Calcd.(%) Found (%) | IR (cm⁻¹) | ¹H-NMR ($CDCl_3$) (δ) |
|---|---|---|---|---|
| I b-7 | 70.0-71.0 ($Et_2O$-iPrOH) | $C_{21}H_{23}N$<br>C, 87.26 (87.15)<br>H, 8.03 (8.01)<br>N, 4.96 (4.84) | ($CHCl_3$)<br>1646, 1485, 1443<br>994, 923 | 7.30-7.36 (2H, m); 7.07-7.12 (6H, m); 6.05 (1H, m); 5.20, 5.26 (2H, m); 3.48 (2H, s); 3.00-3.16, 3.44-3.60 (4H, m); 3.21 (2H, d); 2.76 (4H, m) |
| I b-8 | 205.0-206.0 (MeOH-iPrOH) | $C_{26}H_{27}N \cdot HClO_4$<br>C, 68.53 (68.79)<br>H, 6.20 (6.22)<br>N, 3.22 (3.09)<br>Cl, 7.82 (7.81) | (Nujol)<br>3080, 1476, 1457<br>1113, 1098, 1056<br>753 | 7.04-7.31 (13H, m); 3.44-3.64 (4H, m); 2.69-3.09 (10H, m) |
| I b-9 | oil | ——— | (film)<br>1598, 1482, 1440<br>1354, 1048, 1015<br>766, 748 | 7.35-7.40 (2H, m); 7.07-7.11 (6H, m); 3.54 (2H, s); 3.47-3.58 (2H, m); 2.76 (4H, m); 2.42 (2H, d); 1.05 (1H, m); 0.22, 0.59 (4H, m) |
| I c-1 | 217.0-218.0 (MeOH-iPrOH) | $C_{17}H_{17}N \cdot HCl$<br>C, 70.33 (70.45)<br>H, 6.96 (6.96)<br>N, 4.93 (4.83)<br>Cl 12.42 (12.23) | (Nujol)<br>3270, 1637, 1603<br>1507, 1470, 1050<br>760 | 7.53-7.80 (2H, m); 7.11-7.27 (6H, m); 3.46 (2H, t); 2.94-3.09, 3.34-3.50 (4H, m); 2.78 (1H, brs); 2.40 (2H, t) |
| I c-2 | 108.0-109.0 ($Et_2O$) | $C_{18}H_{19}N \cdot H_2O$<br>C, 80.59 (80.86)<br>H, 7.87 (7.92)<br>N, 5.23 (5.24) | ($CHCl_3$)<br>3620, 3360, 1483<br>1450, 1440, 1271<br>1048 | 7.81-7.86 (2H, m); 7.10-7.35 (6H, m); 3.23 (2H, t); 3.03 (4H, m); 2.24 (2H, t); 2.04 (3H, s) |

Table 4 (No. 6)

| Compd. No. | m. p. (°C) (solvent) | Anal. Calcd. (%) Found (%) | IR (cm⁻¹) | ¹H-NMR ($CDCl_3$) (δ) |
|---|---|---|---|---|
| I c-3 | 153.0-155.0 (MeOH-iPrOH) | $C_{20}H_{21}N \cdot HCl \cdot H_2O$<br>C, 72.65 (72.82)<br>H, 7.39 (7.33)<br>N, 4.55 (4.25)<br>Cl, 10.70 (10.75) | (Nujol)<br>3415, 1650, 1574<br>1505, 1451, 1053<br>983, 927 | 7.81-7.85 (2H, m); 7.11-7.34 (6H, m); 5.82-6.01 (1H, m); 5.05-5.30 (2H, m); 3.25 (2H, t); 3.03 (4H, m); 2.82 (2H, m); 2.28 (2H, t) |
| I c-4 | 142.0-147.0 (MeOH-iPrOH) | $C_{24}H_{23}N \cdot HCl \cdot H_2O$<br>C, 76.00 (75.87)<br>H, 6.97 (6.90)<br>N, 3.87 (3.69)<br>Cl, 9.55 (9.33) | (Nujol)<br>3370, 1575, 1446<br>1423, 1052, 963 | 7.94-7.98 (2H, m); 7.13-7.36 (6H, m); 3.35 (2H, s); 3.29 (2H, t); 3.07 (4H, m); 2.32 (2H, t) |
| I d-1 | 250.0-252.0 (MeOH-iPrOH) | $C_{17}H_{17}N \cdot HCl$<br>C, 75.07 (75.12)<br>H, 6.71 (6.67)<br>N, 5.18 (5.15)<br>Cl, 12.95 (13.05) | (Nujol)<br>2620, 2445, 1554<br>1488, 1463, 1453<br>1329, 953, 920 | 7.44-7.67 (8H, m); 4.62 (4H, s); 3.45 (4H, s); 2.94 (1H, s) |
| I d-3 | 225.0-226.0 (MeOH-iPrOH) | $C_{20}H_{23}N \cdot HClO_4$<br>C, 63.47 (63.57)<br>H, 6.26 (6.40)<br>N, 3.87 (3.71)<br>Cl 9.47 (9.38) | (Nujol)<br>3165, 1480, 1476<br>1454, 1096, 1050<br>888, 745 | 7.26-7.31 (2H, m); 7.06-7.18 (6H, m); 3.86 (4H, s); 3.12 (4H, s); 2.42 (2H, t); 1.43 (2H, m); 0.89 (3H, t) |
| I d-4 | 187.0-188.0 (MeOH-iPrOH) | $C_{25}H_{25}N \cdot HClO_4$<br>C, 68.12 (68.25)<br>H, 5.98 (5.96)<br>N, 3.14 (3.18)<br>Cl, 8.13 (8.06) | (Nujol)<br>3065, 1490, 1455<br>1107, 1050 | 7.07-7.31 (8H, m); 3.90 (4H, s); 3.12 (4H, s); 3.72 (4H, s) |

Table 4 (No. 7)

| Compd. No. | m.p. (°C) (solvent) | Anal. Calcd. (%) Found (%) | IR (cm$^{-1}$) | $^1$H-NMR ($CDCl_3$) (δ) |
|---|---|---|---|---|
| I c-1 | 107.0-108.0 ($CH_2Cl_2$-iPrOH) | $C_{19}H_{21}N$<br>C, 86.42 (86.64)<br>H, 8.07 (8.04)<br>N, 5.34 (5.32) | ($CHCl_3$)<br>1487, 1449, 1094 | 7.25-7.32 (2H, m); 7.03-7.10 (6H, m); 3.88-3.96 (2H, m); 2.93-3.00 (2H, m); 2.54-2.57, 2.60-2.78 (4H, m); 1.89-2.00, 1.43-1.53 (4H, m); 1.28 (1H, brs) |
| I c-2 | 182.0-184.0 (MeOH) | $C_{20}H_{23}N \cdot HClO_4$<br>C, 63.40 (63.57)<br>H, 6.40 (6.40)<br>N, 3.94 (3.71)<br>Cl 9.44 (9.38) | (Nujol)<br>3260, 1465, 1453<br>1105, 1076 | 7.17-7.25 (2H, m); 6.92-7.10 (6H, m); 1.10-4.10 (12H, br); 2.32 (3H, s) |
| I c-3 | 147.0-148.0 (MeOH) | $C_{22}H_{26}N \cdot HClO_4$<br>C, 65.22 (65.42)<br>H, 6.49 (6.49)<br>N, 3.73 (3.47)<br>Cl, 8.73 (8.78) | (Nujol)<br>3240, 1456, 1100<br>1069 | 7.16-7.23 (2H, m); 6.97-7.09 (6H, m); 5.77-5.96 (1H, m); 5.00-5.17 (2H, m); 0.97-4.15 (14H, br) |
| I c-4 | 120.0-128.0 ($Et_2O$-iPrOH) | $C_{26}H_{27}N$<br>C, 88.33 (88.34)<br>H, 7.70 (7.70)<br>N, 4.00 (3.96) | (Nujol)<br>1603, 1495, 1488<br>1450, 1304, 981 | 7.00-7.31 (13H, m); 1.05-4.56 (14H, br) |
| I f-1 | 257.0-263.0 (MeOH-iPrOH) | $C_{19}H_{21}N \cdot HCl$<br>C, 75.96 (76.10)<br>H, 7.50 (7.40)<br>N, 4.70 (4.67)<br>Cl, 11.60 (11.83) | (Nujol)<br>2690, 2600, 1589<br>1563, 1489, 1473<br>1454, 1422, 1387 | 7.25-7.32 (2H, m); 7.03-7.10 (6H, m); 3.88-3.96 (2H, m); 2.93-3.00 (2H, m); 2.54-2.57, 2.60-2.78 (4H, m); 1.89-2.00, 1.43-1.53 (4H, m); 1.28 (1H, brs) |

Table 4 (No. 8)

| Compd. No. | m.p. (°C) (solvent) | Anal. Calcd. (%) Found (%) | IR (cm$^{-1}$) | $^{1}$H-NMR ($CDCl_3$) (δ) |
|---|---|---|---|---|
| I f-2 | 145.0-146.0 ($Et_2O$-iPrOH) | $C_{20}H_{23}N$<br>C, 86.77 (86.59)<br>H, 8.38 (8.36)<br>N, 5.01 (5.05) | ($CHCl_3$)<br>1488, 1475, 1446<br>1131, 1064 | 7.02-7.40 (8H, m); 3.19-3.52 (6H, m); 2.46 (4H, brs); 2.38 (3H, s); 1.73 (2H, m) |
| I f-3 | 207.0-211.0 (iPrOH-$Et_2O$) | $C_{22}H_{27}N \cdot HCl$<br>C, 77.33 (77.28)<br>H, 8.27 (8.25)<br>N, 4.10 (4.10)<br>Cl, 10.37 (10.37) | (Nujol)<br>2410, 1470, 1450<br>748, 743 | 7.02-7.35 (8H, m); 3.23-3.53 (6H, m); 2.39-2.55 (6H, m); 1.57-1.72 (4H, m); 0.94 (3H, t) |
| I f-4 | 122.0-123.0 ($Et_2O$-iPrOH) | $C_{27}H_{29}N$<br>C, 88.08 (88.23)<br>H, 8.01 (7.95)<br>N, 3.77 (3.81) | ($CHCl_3$)<br>1603, 1490, 1477<br>1453 | 7.03-7.33 (13H, m); 3.24-3.54 (6H, m); 2.93 (2H, m); 2.76 (2H, m); 2.45-2.63 (2H, m); 1.73 (2H, m) |
| I g-1 | 116.0-118.0 ($Et_2O$-hexane) | $C_{18}H_{19}N \cdot H_2O$<br>C, 81.02 (80.86)<br>H, 7.92 (7.92)<br>N, 5.25 (5.24) | ($CHCl_3$)<br>3390, 3320, 1579<br>1485, 1080, 839 | 7.81-7.86, 7.61-7.66 (2H, m); 7.11-7.26 (6H, m); 2.89-3.52 (6H, m) 2.80 (1H, d-d); 1.69 (1H, d-d); 1.06 (3H, d) |
| I h-1 | 211.0-222.0 (MeOH-iPrOH) | $C_{19}H_{21}N \cdot HClO_4$<br>C, 62.43 (62.72)<br>H, 6.12 (6.10)<br>N, 4.05 (3.85)<br>Cl, 9.73 (9.75) | (Nujol)<br>3220, 3180, 1596<br>1120, 1105, 1070<br>752 | 7.52-7.82 (2H, m); 7.06-7.14 (6H, m); 3.05-3.64 (5H, m); 2.69-2.83; 2.38-2.53 (2H, m); 1.82-1.98, 1.36-1.51 (3H, m); 1.30 (3H, d) |

*[1]: a solvent for recrystallization

Reference Example 1

Spiro[dibenzosuberane-5,2'-(1-methoxycarbonylpyrrolidine)] (II a-1)

To a solution of 8.77 g of 5-cyanodibenzosuberane in 90 ml of dimethylformamide is added 1.92 g of 60 % sodium hydride under nitrogen atmosphere at -13~-14 °C, and the mixture is stirred at room temperature for 30 minutes. To the mixture is added 10.71 g of 3-tetrahydropyranyloxypropylbromide in ice-methanol bath, and the mixture is stirred at room temperature for 1 hour. The reaction mixture is diluted with ice-water and extracted with toluene to give 15.50 g of 5-cyano-5-tetrahydropyranyloxy propyldibenzosuberane as oily substance. A mixture of the obtained compound and 57.4 g of 86 % potassium hydroxide in 290 ml of ethylene glycol is heated at 158-160 °C for 8 hours. To the reaction mixture are added ice-water and 47.1 g of ammonium chloride, and the mixture is extracted with methylene chloride to give 12.72 g of (5-tetrahydropyranyloxypropyldibenzosuberan-5-yl)carboxyamide. The obtained compound (21.83 g) is dissolved in 330 ml of methanol and cooled in ice-methanol bath (-15~-16 °C). To the reaction mixture are added dropwise sodium methoxide, which was prepared from 4.0 g of metallic sodium and 100 ml of methanol, and a solution of 9.65 g of bromine in 100 ml of methylene chloride at the same temperature for 20 minutes, and the reaction mixture is refluxed for 10 minutes. After removal of methanol under reduced pressure, the residue is extracted with toluene to give 23.66 g of 5-tetrahydropyranyloxypropyl-5-methoxycarbonylaminodibenzosuberane. A mixture of 14.61 g of the obtained compound and 342 mg of p-toluenesulfonic acid monohydrate in 150 ml of methanol is stirred at room temperature for 1 hour. After addition of 5 ml of 2N-$Na_2CO_3$, the reaction mixture is evaporated under reduced pressure to remove methanol. The residue is extracted with toluene to give 11.19 g of 5-hydroxypropyl-5-methoxycarbonyl amino dibenzosuberane. To the mixture of 6.04 g of the obtained compound and 2.86 ml of triethylamine in 60 ml of methylene chloride is added 1.44 ml of methane sulfonyl chloride under cooling. After stirring for 15 minutes, the reaction mixture is diluted with water, extracted with methylene chloride and purified by column chromatography over silica gel to give 6.87 g (Yield : 92%) of 5-(3-methanesulfonyl-oxypropyl)-5-methoxycarbonylamino dibenzosuberane To a mixture of 779 mg of 5-(3-methanesulfonyloxypropyl)-5-methoxycarbonylamino dibenzosuberane in 10 ml of dimethylformamide is added 260 mg of potassium tert-butoxide under cooling, and the mixture is stirred at room temperature for 1 hour. The solution is diluted with water and extracted with toluene. The extract is purified by column chromatography on silica gel to give 572 mg (Yield : 97%) of the compound (II a-1).
IR $\nu$ ($CHCl_3$) : 1670, 1448, 1378 $cm^{-1}$

Reference Example 1 (b)

Another synthetic method of 5-hydroxypropyl-5-methoxycarbonylaminodibenzosuberane

To a solution of 500 mg of 5-cyanodibenzosuberane in 5 ml of dimethylformamide is added 100 mg of 60% sodium hydride under nitrogen atmosphere under cooling, the reaction mixture is stirred at room temperature for 30 minutes. After addition of 0.22 ml of allyl bromide under ice-cooling, the mixture is stirred at room temperature for 30 minutes, diluted with ice-water, acidified with 2N-HCl, and extracted with toluene. The organic layer is washed with water, dried over $MgSO_4$ to give crude product. Purification by lobar column eluting with toluene gives 565 mg (Yield : 96%) of 5-allyl-5-cyanodibenzosuberane as a light yellowish oil. A mixture of 4.0 g of the obtained compound, 18 g of 86 % potassium hydroxide, 6 g of water, and 100 ml of ethylene glycol is heated on an oil bath at 162~164 °C for 2 hours. The reaction mixture is diluted with water, neutralized with hydrochloric acid, and extracted with methylene chloride. The extract is purified by column chromatography on silica gel eluting with a mixture of 15% acetonitrilemethylene chloride. The eluate (3.61 g) is recrystallized from ether-hexane to give 3.25 g (Yield : 76%) of 5-allyldibenzosuberane-5-carbonylamide (mp. 139-141 °C). To a solution of the obtained compound in 96 ml of dry methanol is added sodium methoxide, which is prepared from 1.59 g of metallic sodium and 30 ml of dry methanol at -14 °C. To the reaction mixture is added a solution of 3.70 g of bromine and 20 ml of dry methylene chloride at the same temperature, and the mixture is refluxed for 10 minutes and evaporated under reduced pressure. To the residue is added water, and the solution is extracted with toluene. The extract is purified by column chromatography on silica gel eluting with ethyl acetate-toluene to give 6.95 g (Yield : 98%) of 5-allyl-5-methoxycarbonylaminodibenzosuberane. A solution of 7.55 g of the obtained compound in 76 ml of tetrahydrofuran is added dropwise to a solution of diamyl borane, which is prepared from 11.4 ml of 2-methyl-2-butene and 54 ml of 1.0 mol of borane tetrahydrofuran, at -10~-15 °C, and the mixture is stirred under ice-cooling for 2 hours. To the mixture are added 8.3 ml of 3N-NaOH and 16.6 ml of

hydrogen peroxide, and the mixture is stirred at the same temperature for 1 hour. After removal of the solvent under reduced pressure, the residue is diluted with water and extracted with ether. The extract is purified by column chromatography over silica gel eluting with ethyl acetate-toluene to give 6.04 g of 5-hydroxypropyl-5-methoxy-carbonylamino dibenzosuberane.

Reference Example 2

Spiro[dibenzosuberane-5,3'-(1-benzylsuccinimide)] (II b-1)

To a solution of 4.39 g of 5-cyanodibenzosuberane in 40 ml of dimethylformamide is added 960 mg of sodium hydride in a stream of nitrogen under ice-cooling, and the mixture is stirred at room temperature for 30 minutes, mixed with 2.44 ml of ethyl bromoacetate and stirred at room temperature for 20 minutes. The reaction mixture is diluted with ice-water, acidified with 2N-HCl and extracted with toluene. The extract is washed with water, dried over $MgSO_4$ and purified by column chromatography on silica gel to give 5.81 g (Yield : 95%) of 5-cyano-5-ethoxycarbonylmethyl dibenzosuberane. A solution of 5.31 g of the obtained compound in 16 ml of ether is added to 53 mg of 85 % sulfuric acid under ice-cooling, and the mixture is stirred at the same temperature for 30 minutes. The solution is diluted with water and extracted with methylene chloride. The extract is purified by column chromatography of silica gel to give 5.67 g (Yield : 100%) of 5-ethoxycarbonylmethyldibenzosuberane carboxyamide. To a solution of 5.67 g of the obtained compound in 30 ml of dimethylformamide is added 835 mg of sodium hydride under nitrogen atmosphere under ice-cooling, and the mixture is stirred at room temperature for 30 minutes. To the mixture is added ice-water, and the solution is acidified with 2N-HCl. The resulting crystals are recrystallized from methylene chloride-hexane to give 4.64 g (Yieid : 96%) of spiro(dibenzosuberane-5,3'-succinimide) as white crystals (mp. 222-223 °C). A mixture of 2.0 g of the obtained compound, 1.03 ml of benzylbromide and 1.0 g of potassium carbonate in 20 ml of acetonitrile is refluxed for 40 minutes. After removal of acetonitrile under reduced pressure, the residue is dissolved in toluene and purified by column chromatography of silica gel to give 2.93 g of crude crystals. Recrystallization from methylene chloride-hexane gives 2.55 g (Yield: 96%) of the compound (II b-1) as white crystals. mp.: 160-161 °C

Reference Example 3

Spiro[dibenzosuberane-5,2'-(1-methoxycarbonylazetidine )] (II c-1)

To a solution of 4.39 g of 5-cyanodibenzosuberane in 44 ml of dimethylformamide is added 960 mg of 60% sodium hydride under nitrogen atmosphere at -13~-14 °C, the mixture is stirred at room temperature for 30 minutes. To the mixture is added 4.63 g of ethylene bromohydrin tetrahydropyranyl ether, and the reaction mixture is stirred at room temperature for 1 hour. After removal of dimethylformamide under reduced pressure, to the residue is added water. The solution is extracted with toluene, and the organic layer is washed with water, dried over $MgSO_4$ and purified by column chromatography over silica gel to give 6.97 g (Yield : 100%) of 5-cyano-5-tetrahydropyranyloxy ethyl dibenzosuberane. A mixture of 6.97 g of the obtained compound and 28.6 g of 86% KOH in 140 ml of ethylene glycol is heated on an oil bath at 159-160 °C for 6 hours. To the reaction mixture is added ice, and the mixture is neutralized with HCl, extracted with methylene chloride, washed with water, and dried over $MgSO_4$. The residue is purified by column chromatography of silica gel to give 5.96 g (Yield : 82%) of (5-tetrahydropyranyloxy ethyl dibenzo suberan-5-yl)carbonylamide. To a solution of 5.54 g of the obtained compound in 83 ml of methanol is added a solution of sodium methoxide which is prepared from 1.05 g of metallic sodium and 30 ml of methanol, at -15~-16 °C. To the solution is added dropwise 2.43g of bromine in 24 ml of methylene chloride for 12 minutes, and the mixture is refluxed on an oil bath under heating for 10 minutes. After removal of methanol under reduced pressure, the residue is diluted with water, extracted with methylene chloride, and purified by column chromatography on silica gel to give 6.12 g (Yield : 100%) of 5-tetrahydropyranyl oxy ethyl 5-methoxycarbonylamido dibenzosuberane. A mixture of 6.12 g of the obtained compound and 289 mg of $p$-toluenesulfonic acid monohydrate in 100 ml of methanol is stirred at room temperature for 1.5 hours. To the mixture is added 3 ml of $2N\text{-}Na_2CO_3$, and the mixture is concentrated under reduced pressure to remove methanol and extracted with methylene chloride. The extract is purified by column chromtography on silica gel to give 4.64 g (Yield : 98%) of 5-hydroxyethyl-5-methoxycarbonyl amido dibenzosuberane (mp. 160-161 °C). A mixture of 4.64 g of the obtained compound, 2.29 ml of triethylamine, and 46 ml of methylene chloride is cooled with ice-water, to the mixture is added 1.15 ml of methanesulfonyl chloride. The mixture is stirred for 15 minutes and extracted with water. The extract is

washed with water, dried and purified by column chromatography on silica gel to give 5.43 g (Yield : 94%) of 5-methanesulfonyl oxy ethyl-5-methoxycarbonylamino dibenzosuberane. A solution of 4.87 g of the obtained compound in 50 ml of dimethylformamide is cooled with ice-water. To the mixture is added 1.82 g of potassium tert-butoxide, and the mixture is stirred at room temperature for 1 hour. To the mixture is added water, and the solution is extracted with toluene. The extract is purified by column chromatography of silica gel to give 3.39 g of crude product. Recrystallization from methylene chloride-ether gives 3.01 g (Yield : 82%) of the compound (II c-1) (mp. 135-136 °C).

Reference Example 4

Spiro[dibenzosuberane-5,3'-(1-methoxycarbonylazetidine)] (II d-1)

To a solution of 3.29 g of 5-cyanodibenzosuberane in 33 ml of dimethylformamide under cooling in ice-methanol bath (-13~-14 °C) under nitrogen atmosphere is added 720 mg of 60% sodium hydride, and the mixture is stirred at room temperature for 30 minutes and cooled in ice-methanol bath. To the mixture is added 2.82 g of benzylchloromethyl ether, and the mixture is stirred at room temperature for 30 minutes. After removal of dimethylformamide under reduced pressure, to the residue is added water. The solution is extracted with toluene, and the organic layer is washed with water and dried over $MgSO_4$. The residue is purified by column chromatography on silica gel to give 5.24 g (Yield : 100%) of 5-cyano-5-benzyloxymethyl dibenzosuberane. A solution of 5.24 g of the obtained compound in 120 ml of ethanol is hydrogenated at 70 °C for 2 hours in the presence of Raney® nickel at an initial pressure of 100 atm. After filtration of the catalysis, the solution is concentrated, and the residue is subjected to column chromatography on silica gel to give 4.21 g (Yield : 82%) of (5-benzyl-oxymethyl dibenzosuberan-5-yl)-methylamine. To a solution of 4.20 g of the obtained compound in 42 ml of acetonitrile are added 1.69 g of $K_2CO_3$ and 104 ml of chloromethyl carbonate under ice-cooling, and the mixture is stirred at room temperature for 1.5 hours. The reaction mixture is purified by column chromatography over silica gel to give 4.48 g (Yield : 91%) of 5-benzyloxymethyl-5-methoxycarbonylaminomethyl dibenzosuberane. A mixture of 4.47 g of the obtained compound, 6.0 ml of anisole and 50 ml of nitromethane in 50 ml of methylene chloride is cooled with ice-water, mixed with 4.44 g of aluminium chloride, and stirred at the same temperature for 1 hour. The reaction mixture is diluted with ice-water, and extracted with methylene chloride. The extract is purified by column chromatography on silica gel to give 3.71 g (Yield : 100%) of 5-hydroxymethyl-5-methoxycarbonyl aminomethyl dibenzosuberane. To a mixture of 3.71 g of the obtained compound, 1.70 ml of triethylamine in 50 ml of methylene chloride is added 0.86 ml of methanesulfonyl chloride under ice-cooling. The mixture is stirred for 15 minutes, and extracted with water, and the organic layer is washed with water, and dried. The product is purified by column chromatograpy of silica gel to give 4.46 g (Yield : 100%) of 5-methanesulfonyloxy methyl-5-methoxycarbonyl aminomethyl dibenzosuberane. To a solution of 1.48 g of the obtained compound in 20 ml of dimethylformamide is added 512 mg of potassium tert-butoxide under ice-cooling, and the mixture is stirred at room temperature for 45 minutes. To the mixture is added water, and the solution is extracted with toluene. The organic layer is washed with water, dried and subjected to column chromatography over silica gel to give 936 mg of crude crystals. Recrystallization from methylene chloride-ether gives 876 mg (Yield : 79 %) of the compound (II d-1) as white crystals.
mp. 149-150 °C

Reference Example 5

Spiro[dibenzosuberane-5,2'-(1-methoxycarbonylpiperidine)] (II e-1)

To a solution of 4.39 g of 5-cyanodibenzosuberane in 40 ml of dimethylformamide is added 960 mg of 60% sodium hydride under nitrogen atmosphere under cooling, and the mixture is stirred at room temperature for 30 minutes. To the mixture is added 4.95 g of 4-bromobutanol trimethyl silylether, and the mixture is stirred at room temperature for 1 hour. After removal of dimethylformamide under reduced pressure, to the residue are added ether, ice-water, and 10 ml of 2N-$H_2SO_4$, and the mixture is stirred at room temperature for 40 minutes. The ether layer is washed with water and purified by column chromatography of silica gel to give 5.30 g (Yield : 91%) of 5-cyano-5-(4-hydroxybutyl)dibenzosuberane as colorless oily substance. A mixture of 5.30 g of the obtained compound, 21.2 g of 86 % of KOH, and 100 ml of ethylene glycol is heated on an oil bath at 158-160 °C for 2.5 hours. The mixture is mixed with ice, neutralized with hydrochloride, extracted with methylene chloride. The organic layer is washed with water, and dried. The residue is subjected to column chromatography of silica gel to give 5.11 g (Yield: 91%) of

[5-(4-hydroxybutyl)dibenzosuberan-5-yl]carbonylamide as white powder. A solution of 5.11 g of the obtained compound in 77 ml of methanol is cooled in ice-methanol bath (-13~-15°C), and to the mixture are added dropwise a solution of sodium methoxide, which is prepared from 1.14 g of metallic sodium and 30 ml of dry methanol, and 2.64 g of bromine in 26 ml of methylene chloride for 13 minutes. The reaction mixture is refluxed on an oil bath under heating for 10 minutes. After removal of methanol under reduced pressure, to the residue is added water. The solution is extracted with methylene chloride, washed with water, dried and subjected to column chromatography of silica gel to give 5.21 g (Yield : 93%) of 5-(4-hydroxybutyl)-5-methoxycarbonyl aminodibenzosuberane. A mixture of 5.21 g of the obtained compound and 2.80 ml of triethylamine in 50 ml of methylene chloride is cooled with ice-water, and 1.2 ml of methanesulfonyl chloride is added thereto. The reaction mixture is stirred for 20 minutes and extracted with water. The organic layer is washed with water, dried and subjected to column chromatography of silica gel to give 6.41 g (Yield : 100%) of 5-(4-methanesulfonyloxybutyl)-5-methoxycarbonylaminodibenzosuberane. To a solution of 6.40 g of the obtained compound in 64 ml of dimethylformamide is added 2.06 g of potassium tert-butoxide under ice-cooling, and the mixture is stirred at room temperature for 1 hour. The solution is mixed with water, acidified with N-HCl, and extracted with toluene. The organic layer is washed with water, dried and subjected to column chromatography of silica gel to give 4.93 g (Yield : 100%) of the objective compound (II e-1).
IR $\nu$ ($CHCl_3$) : 1680, 1450, 1380 $cm^{-1}$

Reference Example 6

5-Cyano-5-(3-chloropropyl)dibenzosuberane (II f-1)

To a solution of 4.39 g of 5-cyanodibenzosuberane in 44 ml of dimethylformamide is added 960 mg of 60 % sodium hydride under nitrogen atmosphere under ice-cooling. The reaction mixture is stirred at room temperature for 30 minutes and cooled with ice-water. To the mixture is added 2.37 ml of 1-bromo-3-chloropropane, and the mixture is stirred at room temperature for 1 hour. The reaction mixture is mixed with water, acidified with 10 ml of 2N-HCl, and extracted with toluene. The organic layer is washed with water, dried and subjected to column chromatgraphy of silica gel to give 5.93 g (Yield : 100 %) of the compound (II f-1) as a light yellowish oily substance.

Reference Example 7

Spiro[dibenzosuberane-5,2'-(1-methoxycarbonyl-4-methylpyrrolidine)] (II g-1)

To a mixture of 2.14 g of 5-allyl-5-methoxycarbonyl aminodibenzosuberane, which is prepared in Reference Example 1 as an intermediate and 1.92 g of potassium carbonate in 40 ml of methylene chloride is added 1.77 g of iodine, and the mixture is stirred at room temperature for 2.5 hours. After decomposition of the excess iodine by the addition of $Na_2S_2O_3 \cdot 5H_2O$, the mixture is extracted with methylene choride and purified by column chromatography on silica gel to give 2.69 g of spiro[dibenzosuberane-5,2'-(1-methoxycarbonyl-4-iodomethylazetidine )] as a colorless oily substance. A mixture of 2.69 g of the obtained compound, 2.35 g of tributyltin iodide, 204 mg of 2,2'-azobisisobutyronitrile, and 50 ml of benzene is refluxed on an oil bath under heating for 1.5 hours, washed with 2N sodium carbonate, and purified by column chromatography over silica gel to give 1.42 g (Yield : 74%) of the objective compound (II g-1).

Reference Example 8

Spiro[dibenzosuberane-5,2'-(5-phenylthiomethyl)pyrrolidine] (II h-1)

To a solution of 3.29 g of 5-cyanodibenzosuberane in 33 ml of dimethylformamide is added 720 mg of 60% sodium hydride in ice-methanol bath (-13~-14°C) under nitrogen atmosphere, and the mixture is stirred at room temperature for 30 minutes, and cooled in ice-methanol bath. To the mixture is added 1.83 ml of 4-bromo-1-butene, and the mixture is stirred at room temperature for 1 hour. The reaction mixture is diluted with water, acidified with 2N-hydrochloride, and extracted with toluene. The organic layer is washed with water, dried and subjected to column chromatography of silica gel to give 4.03 g (Yield : 98%) of 5-cyano-5-(3-butenyl)dibenzosuberane. A mixture of 4.03 g of the obtained compound and 16 g of 86% KOH in 80 ml of ethylene glycol is stirred at 162-164 °C for 6 hours, extracted with methylene chloride, and purified by column chromatography over silica gel to give 3.68 g ( Yield : 86%) of [5-(3-butenyl)-dibenzosuberan-5-yl]carbonylamide as a colorless oil. To a solution of 3.68 g of the obtained compound in 50 ml of methanol is added sodium methoxide, which is prepared from 869 mg of metallic sodium and 30 ml of dry methanol, under cooling with ice-methanol bath (-15~-16 °C). To the reaction mixture is added dropwise a solution of 2.11 g of bromine in 10 ml of methylene chloride at the same temperature for 10 minutes, and the mixture is refluxed on an oil bath for 10 minutes. After removal of methanol, the residue is extracted with methylene chloride. The organic layer is washed with water, and purified by column chromatography of silica gel to give 3.32 g (Yield : 82%) of 5-(3-butenyl)-5-methoxy-carbonylamino dibenzosuberane (mp. 149-150 °C). A mixture of 3.32 g of the obtained compound and 3.37 g of 86% KOH in 33 ml of n-butanol is refluxed on for 15 hours. After removal of n-butanol, the mixture is extracted with methylene chloride and purified by column chromatography of silica gel to give 2.92 g (Yield : 93%) of 5-(3-butenyl)-5-aminodibenzosuberane hydrochloride (mp. 207-219 °C) To a mixture of 1.26 g of phenyldisulfide and 0.58 ml of sulfonyl chloride in 60 ml of acetonitrile is added 3.15 g of the obtained compound under ice-cooling, and the mixture is refluxed for 15 minutes and further 30 minutes at room temperature. To the solution are added 6.33 g of potassium carbonate and 6.3 g of sodium iodide and the mixture is refluxed for 1 hour. After removal of the solvent, the product is extracted with methylene chloride, washed with water, dried and purified by column chromatography over silica gel to give 2.78g (Yield : 71%) of spiro-(dibenzosuberane-5,2'-(5-phenylthiomethyl)pyrrolidine] (II h-1) as a light yellowish oil.

IR ν ($CHCl_3$) : 3374, 1581, 1477, 1436, 1087 $cm^{-1}$

Reference Example 9

Spiro[3-bromodibenzosuberane-5,2'-(1-trifluoroacetylpyrrolidine)] (II a-4)

3-Bromo-5-cyanodibenzosuberane 6.25 g is reacted with 5.58 g of 3-tetrahydropyranyloxypropylbromide, and treated in the same manner as Reference Example 1 to give 6.60 g (Yield: 64.5%) of 3-bromo-5-tetrahydropyranyloxypropyl-5-methoxycarbonylamino dibenzosuberane. A mixture of 8.28 g of the compound and 5.55 g of 86% potassium hydroxide in 83 ml of n-butanol is refluxed for 15 hours. After removal of n-butanol under reduced pressure, the residue is dissolved in a mixture of 50 ml of methanol and 30 ml of water and stirred with 15 ml of c.HCl for 1 hour. To the mixture is added 3.4 g of sodium hydroxide, and the reaction mixture is concentrated under reduced pressure and extracted with methylene chloride to give 5.91 g (Yield : 100%) of 5-amino-3-bromo-5-(3-hydroxypropyl)-dibenzosuberane. A mixture of 5.90 g of the compound, 5.21 g of triethylamine, and 60 ml of methylene chloride is cooled with ice-water, mixed with 5.28 ml of trifluoroacetic anhydride and stirred for 15 minutes. To the reaction mixture is added 20 ml of 2N sodium hydroxide. After stirring at room temperature for 30 minutes, the reaction mixture is diluted with water, neutralized with 2N-hydrochloride, and extracted with methylene chloride. The organic layer is washed with water, dried over $MgSO_4$, and purified by column chromatography on silica gel eluting with 10% acetonitrile-methylene chloride to give 7.19 g (Yield : 96%) of 3-bromo-5-(3-hydroxypropyl)-5-trifluoroacetylaminodibenzosuberane. A mixture of 7.19 g of the obtained compound, 5 ml of triethylamine, and 72 ml of ethylene chloride is cooled with ice-water, mixed with 3.05 ml of methanesulfonyl chloride, and stirred for 15 minutes. After removal of methylene chloride under reduced pressure, the residue is dissolved in 50 ml of dimethylformamide. To the reaction mixture is added 9.9 g of anhydrous potassium carbonate, and the mixture is stirred at room temperature for 1.5 hours. The reaction mixture is diluted with water, and extracted with toluene. The extract is subjected to column chromatography on silica gel to give 7.23 g (Yield : 100%) of the objective compound (II a-4) as a colorless oil.

IR ν ($CHCl_3$) : 1696, 1483, 1436, 1243, 1195, 1141 $cm^{-1}$

Reference Literature:

Synthesis of the starting material (5-cyanodibenzosuberane)

K.Ackermann, J.Chapuis, D.E.Horning, G.Lacasse, and J.M.Muchowski, Can. J. Chem., 47 4327 (1969)

## Evaluation of biological activity

Binding test against PCP (Phencylidine) receptor Experiment

After the decapitation of rats (male, Slc-Wistar rat, 10 15 weeks age), the cortex tissues was rapidly removed and each was weighed. The tissue was homogenized with ten-fold amount of ice-cold 50mM Tris-HCl buffer (pH7.4), and centrifuged at 40,000 × g for 10 minutes. This procedure is repeated 3 times. Then the obtained sample was freeze-dried in liquid nitrogen and preserved at -80 °C On the test day, the sample was thawed at room temperature and centrifuged at 40,000 × g for 10 minutes, the pellets were then suspended in 5mM Tris-HCl buffer and used as the receptor preparation (0.2mg protein/ml). The preparation was added to the mixture containing the labelled ligand ($^3$H-TCP, 5mM) and the test drug, and the incubation was carried out (25 °C 30 minutes). The reaction was stopped by rapid filtering through Whatman GF/C filter and the filtrate was washed. Radioactivity on filters was determined by liquid scintillation counter and Ki value was calculated. The Whatmann GF/C filter was soaked in 0.05 % polyethyleneimine.
The test results were shown in Table 5.
(Literature : Vignon, J. et al., Brain R.,280; 194-197 (1983))

Table 5

| Test Compound | Ki( μ M) PCP |
|---|---|
| I a-1 | 0. 047 |
| I a-3 | 0. 62 |
| I a-7 | 0. 031 |
| I a-8 | 0. 51 |
| I a-9 | 0. 77 |
| I a-10 | 0. 25 |
| I a-11 | >0. 71 |
| I a-13 | 0. 023 |
| I a-14 | 0. 54 |

**Claims**
**Claims for the following Contracting States : AT, BE, CH, DE, DK, FR, GB, IT, LI, LU, NL, SE**

**1.** A compound represented by the formula (I):

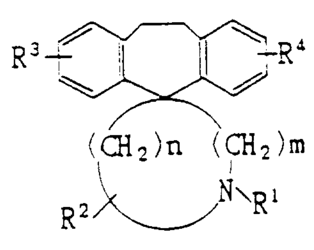

( I )

wherein $R^1$ is hydrogen, alkyl, alkenyl, or aralkyl; $R^2$ is hydrogen or alkyl; $R^3$ and $R^4$ which are the same or different are hydrogen, alkyl, alkoxy, or halogen; m is 0 or 1; n is an integer of 1-4, or a pharmaceutically acceptable acid addition salt thereof, excluding spiro(dibenzosuberane-5,3'-piperidine).

**2.** The compound claimed in claim 1, wherein m is 0.

**3.** The compound claimed in claim 1, wherein m is 1.

**4.** The compound claimed in claim 1, wherein m is 0 and n is 3.

**5.** The compound claimed in claim 1, wherein m is 0 and n is 2.

**6.** The compound claimed in claim 1, wherein m is 0 and n is 4.

**7.** The compound claimed in claim 4, wherein $R^1$, $R^2$, and $R^3$ each is hydrogen, $R^4$ is hydrogen, alkyl, or halogen.

**8.** The compound claimed in claim 1, namely, spiro(dibenzosuberane-5,2'-pyrrolidine).

**9.** The compound claimed in claim 1, namely, spiro(3-methyldibenzosuberane-5,2'-pyrrolidine).

**10.** The compound claimed in claim 1, namely, spiro(3-chlorodibenzosuberane-5,2'-pyrrolidine).

**11.** A pharmaceutical composition comprising a pharmacologically effective amount of the compound claimed in claim 1 together with a carrier, diluent, and/or excipient.

**12.** A compound claimed in any one of claims 1 to 10 for use in the protection against ischemic encephalopathy, the prevention of necrosis of brain cells and the prevention and treatment of Alzheimer's disease.

**13.** A method for producing a compound of the formula I as defined in any one of claims 1-10 which comprises

a) subjecting a compound of the formula II

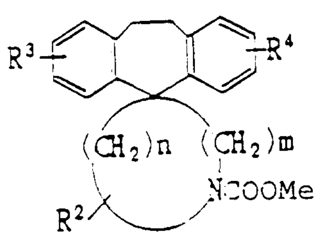

(II)

to reduction to give a compound of the formula I wherein $R^1$ is Me; or

b) subjecting the compound of the formula (II) to hydrolysis and then to decarboxylation to obtain a compound of the formula I wherein $R^1$ is hydrogen and optionally further treating the obtained product in a per se known manner to introduce a group $R^1$ which is different from Me.

**14.** Use of a compound as defined in any one of claims 1 to 10 including the compound spiro-(dibenzosuberane-5,3'piperidine) for the preparation of a pharmaceutical composition useful in the treatment and prevention of neurodegenerative diseases.

**Claims for the following Contracting State : ES**

1. A method for producing a compound represented by the formula (I) :

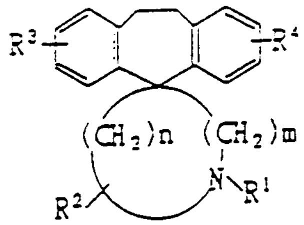

( I )

wherein $R^1$ is hydrogen, alkyl, alkenyl, or aralkyl; $R^2$ is hydrogen or alkyl; $R^3$ and $R^4$ which are the same or different are hydrogen, alkyl, alkoxy, or halogen; m is 0 or 1; n is an integer or 1-4, or a pharmaceutically acceptable acid addition salt thereof, excluding spiro(dibenzosuberane-5,3'-piperidine) which comprises

a) subjecting a compound of the formula II

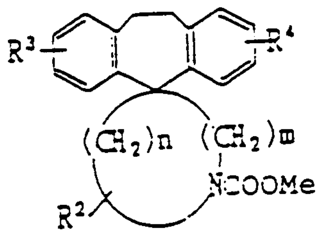

(II)

to reduction to give a compound of the formula I wherein $R^1$ is Me; or

b) subjecting the compound of the formula (II) to hydrolysis and then to decarboxylation to obtain a compound of the formula I wherein $R^1$ is hydrogen and optionally further treating the obtained product in a per se known manner to introduce a group $R^1$ which is different from Me.

2. The method claimed in claim 1 for producing a compound of the formula I, wherein m is 0.

3. The method claimed in claim 1 for producing a compound of the formula I, wherein m is 1.

4. The method claimed in claim 1 for producing a compound of the formula I, wherein m is 0 and n is 3.

5. The method claimed in claim 1 for producing a compound of the formula I, wherein m is 0 and n is 2.

6. The method claimed in claim 1 for producing a compound of the formula I, wherein m is 0 and n is 4.

7. The method claimed in claim 4 for producing a compound of the formula I, wherein $R^1$, $R^2$, and $R^3$ each is hydrogen, $R^4$ is hydrogen, alkyl, or halogen.

8. The method claimed in claim 1 for producing the compound spiro(dibenzosuberane-5,2'-pyrrolidine).

9. The method claimed in claim 1 for producing the compound spiro(3-methyldibenzosuberane-5,2'-pyrrolidine).

10. The method claimed in claim 1 for producing the compound spiro(3-chlorodibenzosuberane-5,2'-pyrrolidine).

11. A method for producing a pharmaceutical composition comprising combining a pharmacologically effective amount of the compound claimed in claim 1 with a carrier, diluent, and/or excipient.

12. A method for the preparation of a pharmaceutical composition for the treatment and prevention of neurodegenerative diseases comprising combining a compound as defined in claim 1 including spiro-(dibenzosuberane-5,3'piperidine) with a carrier, diluent and/or excipient.

**Claims for the following Contracting State : GR**

1. A compound represented by the formula (I):

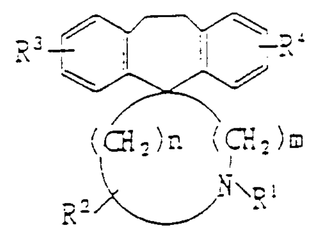
( I )

wherein $R^1$ is hydrogen, alkyl, alkenyl, or aralkyl; $R^2$ is hydrogen or alkyl; $R^3$ and $R^4$ which are the same or different are hydrogen, alkyl, alkoxy, or halogen; m is 0 or 1; n is an integer of 1-4, or a pharmaceutically acceptable acid addition salt thereof, excluding spiro(dibenzosuberane-5,3'-piperidine)

2. The compound claimed in claim 1, wherein m is 0.

3. The compound claimed in claim 1, wherein m is 1.

4. The compound claimed in claim 1, wherein m is 0 and n is 3.

5. The compound claimed in claim 1, wherein m is 0 and n is 2.

6. The compound claimed in claim 1, wherein m is 0 and n is 4.

7. The compound claimed in claim 4, wherein $R^1$, $R^2$, and $R^3$ each is hydrogen $R^4$ is hydrogen, alkyl, or halogen.

8. The compound claimed in claim 1, namely, spiro(dibenzosuberane-5,2'-pyrrolidine).

9. The compound claimed in claim 1, namely, spiro(3-methyldibenzosuberane-5,2'-pyrrolidine).

10. The compound claimed in claim 1, namely, spiro(3-chlorodibenzosuberane-5,2'-pyrrolidine).

11. A method for producing a pharmaceutical composition comprising combining a pharmacologically effective amount of the compound claimed in claim 1 with a carrier, diluent and/or excipient.

12. A method for producing a compound of the formula I as defined in any one of claims 1-10 which comprises
    a) subjecting a compound of the formula II

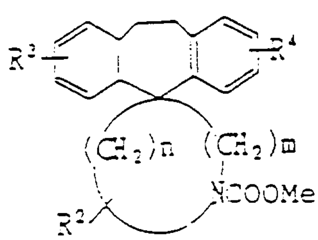
(II)

to reduction to give a compound of the formula I wherein $R^1$ is Me; or
b) subjecting the compound of the formula (II) to hydrolysis and then to decarboxylation to obtain a compound of the formula I wherein $R^1$ is hydrogen and optionally further treating the obtained product in a per se known manner to introduce a group $R^1$ which is different from Me.

**13.** A method for the preparation of a pharmaceutical composition for the treatment and prevention of neurodegenerative diseases comprising combining a compound as defined in claim 1 including spiro-(dibenzosuberane-5,3'piperidine) with a carrier, diluent and/or excipient.

**Patentansprüche**
**Patentansprüche für folgende Vertragsstaaten : AT, BE, CH, DE, DK, FR, GB, IT, LI, LU, NL, SE**

**1.** Verbindung mit der Formel (I)

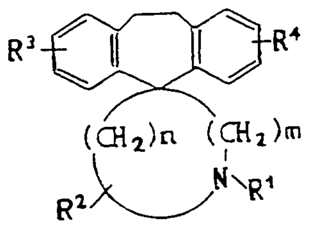

(I)

wobei $R^1$ ein Wasserstoffatom, ein Alkyl-, Alkenyl- oder Aralkylrest ist, $R^2$ ein Wasserstoffatom oder ein Alkylrest ist, $R^3$ und $R^4$, die gleich oder unterschiedlich sind, ein Wasserstoffatom, einen Alkylrest, einen Alkoxyrest oder ein Halogenatom bedeuten, m den Wert 0 oder 1 hat, und n eine ganze Zahl im Wert von 1 bis 4 ist, oder ein pharmazeutisch verträgliches Säureadditionssalz davon, außer Spiro-(dibenzosuberan-5,3'-piperidin).

**2.** Verbindung nach Anspruch 1, wobei m den Wert 0 hat.

**3.** Verbindung nach Anspruch 1, wobei m den Wert 1 hat.

**4.** Verbindung nach Anspruch 1, wobei m den Wert 0 und n den Wert 3 hat.

**5.** Verbindung nach Anspruch 1, wobei m den Wert 0 und n den Wert 2 hat.

**6.** Verbindung nach Anspruch 1, wobei m den Wert 0 und n den Wert 4 hat.

**7.** Verbindung nach Anspruch 4, wobei $R^1$, $R^2$ und $R^3$ jeweils Wasserstoffatome sind und $R^4$ ein Wasserstoffatom, ein Alkylrest oder ein Halogenatom ist.

**8.** Verbindung nach Anspruch 1, die Spiro(dibenzosuberan-5,2'-pyrrolidin) ist.

**9.** Verbindung nach Anspruch 1, die Spiro(3-methyldibenzosuberan-5,2'-pyrrolidin) ist.

**10.** Verbindung nach Anspruch 1, die Spiro(3-chlordibenzosuberan-5,2'-pyrrolidin) ist.

**11.** Arzneimittel, umfassend eine pharmakologisch wirksame Menge der Verbindung nach Anspruch 1 zusammen mit einem Träger, Verdünnungsmittel und/oder Excipienten.

**12.** Verbindung nach einem der Ansprüche 1 bis 10 für die Verwendung zum Schutz gegen ischämische Enzephalopathie, zur Verhütung der Nekrose von Gehirnzellen und zur Verhütung und Behandlung der Alzheimer-Krankheit.

**13.** Verfahren zur Herstellung einer Verbindung der Formel (I) nach einem der Ansprüche 1 bis 10, umfassend

a) Reduktion einer Verbindung der Formel (II)

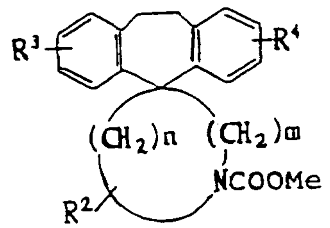

(II)

zu einer Verbindung der Formel (I), in der $R^1$ Me ist, oder

b) Hydrolyse der Verbindung der Formel (II) und danach Decarboxylierung zu einer Verbindung der Formel (I), in der $R^1$ Wasserstoff ist, und gegebenenfalls eine weitere Behandlung des erhaltenen Produkts nach bekannten Verfahren zur Einführung eines Restes $R^1$, der sich von Me unterscheidet.

**14.** Verwendung einer Verbindung nach einem der Ansprüche 1 bis 10 einschließlich der Verbindung Spiro-(dibenzosuberan-5,3'-piperidin) zur Herstellung eines Arzneimittels, das zur Behandlung und zur Verhütung von neurodegenerativen Erkrankungen nützlich ist.

**Patentansprüche für folgenden Vertragsstaat : ES**

**1.** Verfahren zur Herstellung einer Verbindung mit der Formel (I)

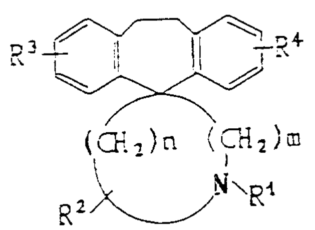

(I)

wobei $R^1$ ein Wasserstoffatom, ein Alkyl-, Alkenyl- oder Aralkylrest ist, $R^2$ ein Wasserstoffatom oder ein Alkylrest ist, $R^3$ und $R^4$, die gleich oder unterschiedlich sind, ein Wasserstoffatom, einen Alkylrest, einen Alkoxyrest oder ein Halogenatom bedeuten, m den Wert 0 oder 1 hat, und n eine ganze Zahl im Wert von 1 bis 4 ist, oder ein pharmazeutisch verträgliches Säureadditionssalz davon, außer Spiro-(dibenzosuberan-5,3'-piperidin, umfassend

a) Reduktion einer Verbindung der Formel(II)

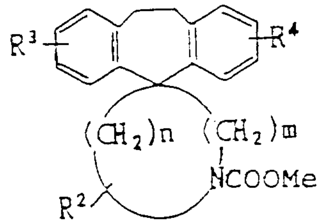

(II)

zu einer Verbindung der Formel (I), in der $R^1$ Me ist, oder

b) Hydrolyse der Verbindung der Formel (II) und danach Decarboxylierung zu einer Verbindung der Formel (I), in der $R^1$ Wasserstoff ist, und gegebenenfalls eine weitere Behandlung des erhaltenen Produkts nach bekannten Verfahren zur Einführung eines Restes $R^1$, der sich von Me unterscheidet.

2. Verfahren von Anspruch 1 zur Herstellung einer Verbindung mit der Formel (I), wobei m den Wert 0 hat.

3. Verfahren von Anspruch 1 zur Herstellung einer Verbindung mit der Formel (I), wobei m den Wert 1 hat.

4. Verfahren von Anspruch 1 zur Herstellung einer Verbindung mit der Formel (I), wobei m den Wert 0 und n den Wert 3 hat.

5. Verfahren von Anspruch 1 zur Herstellung einer Verbindung mit der Formel (I), wobei m den Wert 0 und n den Wert 2 hat.

6. Verfahren von Anspruch 1 zur Herstellung einer Verbindung mit der Formel (I), wobei m den Wert 0 und n den Wert 4 hat.

7. Verfahren nach Anspruch 4 zur Herstellung einer Verbindung mit der Formel (I), wobei $R^1$, $R^2$ und $R^3$ jeweils Wasserstoffatome sind und $R^4$ ein Wasserstoffatom, ein Alkylrest oder ein Halogenatom ist.

8. Verfahren nach Anspruch 1 zur Herstellung der Verbindung Spiro(dibenzosuberan-5,2'-pyrrolidin).

9. Verfahren nach Anspruch 1 zur Herstellung der Verbindung Spiro(3-methyldibenzosuberan-5,2'-pyrrolidin).

10. Verfahren nach Anspruch 1 zur Herstellung der Verbindung Spiro(3-chlordibenzosuberan-5,2'-pyrrolidin).

11. Verfahren zur Herstellung eines Arzneimittels, umfassend das Kombinieren einer pharmakologisch wirksamen Menge der Verbindung nach Anspruch 1 mit einem Träger, Verdünnungsmittel und/oder Excipienten.

12. Verfahren zur Herstellung eines Arzneimittels zur Behandlung und zur Verhütung von neurodegenerativen Erkrankungen, umfassend das Kombinieren einer Verbindung nach Anspruch 1 einschließlich der Verbindung Spiro(dibenzosuberan-5,3'-piperidin), mit einem Träger, Verdünnungsmittel und/oder Excipienten.

**Patentansprüche für folgenden Vertragsstaat : GR**

1. Verbindung mit der Formel (I)

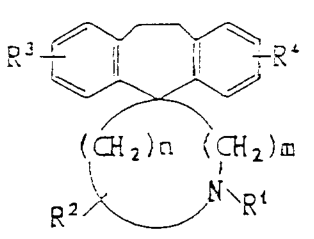

(I)

wobei $R^1$ ein Wasserstoffatom, ein Alkyl-, Alkenyl- oder Aralkylrest ist, $R^2$ ein Wasserstoffatom oder ein Alkylrest ist, $R^3$ und $R^4$, die gleich oder unterschiedlich sind, ein Wasserstoffatom, einen Alkylrest, einen Alkoxyrest oder ein Halogenatom bedeuten, m den Wert 0 oder 1 hat, und n eine ganze Zahl im Wert von 1 bis 4 ist, oder ein pharmazeutisch verträgliches Säureadditionssalz davon, außer Spiro-(dibenzosuberan-5,3'-piperidin).

2. Verbindung nach Anspruch 1, wobei m den Wert 0 hat.

3. Verbindung nach Anspruch 1, wobei m den Wert 1 hat.

**4.** Verbindung nach Anspruch 1, wobei m den Wert 0 und n den Wert 3 hat.

**5.** Verbindung nach Anspruch 1, wobei m den Wert 0 und n den Wert 2 hat.

**6.** Verbindung nach Anspruch 1, wobei m den Wert 0 und n den Wert 4 hat.

**7.** Verbindung nach Anspruch 4, wobei $R^1$, $R^2$ und $R^3$ jeweils Wasserstoffatome sind und $R^4$ ein Wasserstoffatom, ein Alkylrest oder ein Halogenatom ist.

**8.** Verbindung nach Anspruch 1, die Spiro(dibenzosuberan-5,2'-pyrrolidin) ist.

**9.** Verbindung nach Anspruch 1, die Spiro(3-methyldibenzosuberan-5,2'-pyrrolidin) ist.

**10.** Verbindung nach Anspruch 1, die Spiro(3-chlordibenzosuberan-5,2'-pyrrolidin) ist.

**11.** Verfahren zur Herstellung eines Arzneimittels, umfassend das Kombinieren einer pharmakologisch wirksamen Menge der Verbindung nach Anspruch 1 mit einem Träger, Verdünnungsmittel und/oder Excipienten.

**12.** Verfahren zur Herstellung einer Verbindung der Formel (I) nach einem der Ansprüche 1 bis 10, umfassend

a) Reduktion einer Verbindung der Formel (II)

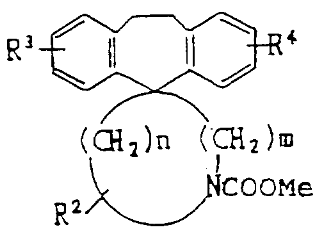

(II)

zu einer Verbindung der Formel (I), in der $R^1$ Me ist, oder

b) Hydrolyse der Verbindung der Formel (II) und danach Decarboxylierung zu einer Verbindung der Formel (I), in der $R^1$ Wasserstoff ist, und gegebenenfalls eine weitere Behandlung des erhaltenen Produkts nach bekannten Verfahren zur Einführung eines Restes $R^1$, der sich von Me unterscheidet.

**13.** Verfahren zur Herstellung eines Arzneimittels zur Behandlung und zur Verhütung von neurodegenerativen Erkrankungen, umfassend das Kombinieren einer Verbindung nach Anspruch 1 einschließlich der Verbindung Spiro(dibenzosuberan-5,3'-piperidin), mit einem Träger, Verdünnungsmittel und/oder Excipienten.

**Revendications**

**Revendications pour les Etats contractants suivants : AT, BE, CH, DE, DK, FR, GB, IT, LI, LU, NL, SE**

**1.** Composé représenté par la formule (I) :

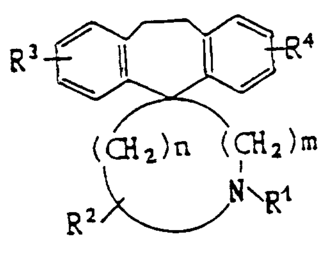

( I )

dans laquelle $R^1$ est un hydrogène, un alkyle, un alcényle ou un aralkyle ; $R^2$ est un hydrogène ou un alkyle ; $R^3$ et $R^4$ qui sont identiques ou différents, sont un hydrogène, un alkyle, un alcoxy ou un halogène ; m est 0 ou 1 ; n est un entier de 1 à 4, ou un sel d'addition d'acide pharmaceutiquement acceptable de celui-ci, à l'exclusion de la spiro(dibenzosubérane-5,3'-pipéridine).

**2.** Composé selon la revendication 1, où m est 0.

**3.** Composé selon la revendication 1, où m est 1.

**4.** Composé selon la revendication 1, où m est 0 et n est 3.

**5.** Composé selon la revendication 1, où m est 0 et n est 2.

**6.** Composé selon la revendication 1, où m est 0 et n est 4.

**7.** Composé selon la revendication 4, où $R^1$, $R^2$ et $R^3$ sont chacun un hydrogène et $R^4$ est un hydrogène, un alkyle ou un halogène.

**8.** Composé selon la revendication 1, qui est la spiro(dibenzosubérane-5,2'-pyrrolidine).

**9.** Composé selon la revendication 1, qui est la spiro(3-méthyldibenzosubérane-5,2'-pyrrolidine).

**10.** Composé selon la revendication 1, qui est la spiro(3-chlorodibenzosubérane-5,2'-pyrrolidine).

**11.** Composition pharmaceutique comprenant une quantité pharmacologiquement efficace d'un composé selon la revendication 1, avec un véhicule, diluant et/ou excipient.

**12.** Composé selon l'une quelconque des revendications 1 à 10 pour l'utilisation dans la protection contre l'encéphalopathie ischémique, la prévention de la nécrose des cellules cérébrales et la prévention et le traitement de la maladie d'Alzheimer.

**13.** Procédé pour préparer un composé de formule I, tel que défini dans l'une quelconque des revendications 1 à 10, qui consiste à

a) soumettre un composé de formule (II) à une réduction

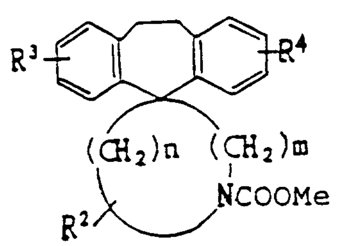

(II)

pour obtenir un composé de formule (I) dans laquelle $R^1$ est Me ; ou

b) soumettre le composé de formule (II) à une hydrolyse puis à une décarboxylation pour obtenir un composé de formule (I) dans laquelle $R^1$ est un hydrogène et, éventuellement, traiter de plus le produit obtenu de façon connue en soi pour introduire un groupe $R^1$ qui est différent de Me.

**14.** Utilisation d'un composé tel que défini dans l'une quelconque des revendications 1 à 10, y compris le composé spiro(dibenzosubérane-5,3'-pipéridine), pour la préparation d'une composition pharmaceutique utile dans le traitement et la prévention des maladies neurodégénératives.

**Revendications pour l'Etat contractant suivant : ES**

**1.** Procédé pour préparer un composé représenté par la formule (I) :

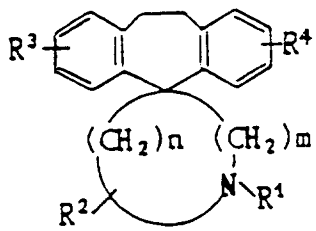

( I )

dans laquelle $R^1$ est un hydrogène, un alkyle, un alcényle ou un aralkyle ; $R^2$ est un hydrogène ou un alkyle ; $R^3$ et $R^4$ qui sont identiques ou différents, sont un hydrogène, un alkyle, un alcoxy ou un halogène ; m est 0 ou 1 : n est un entier de 1 à 4, ou un sel d'addition d'acide pharmaceutiquement acceptable de celui-ci, à l'exclusion de la spiro(dibenzosubérane-5,3'-pipéridine), qui consiste à

a) soumettre un composé de formule (II) à une réduction

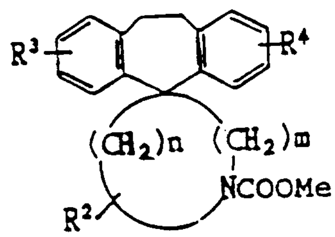

(II)

pour obtenir un composé de formule (I) dans laquelle $R^1$ est Me ; ou

b) soumettre le composé de formule (II) à une hydrolyse puis à une décarboxylation pour obtenir un composé de formule (I) dans laquelle $R^1$ est un hydrogène et, éventuellement, traiter de plus le produit obtenu de façon connue en soi pour introduire un groupe $R^1$ qui est différent de Me.

**2.** Procédé selon la revendication 1 pour préparer un composé de formule (I) où m est 0.

**3.** Procédé selon la revendication 1 pour préparer un composé de formule (I) où m est 1.

**4.** Procédé selon la revendication 1 pour préparer un composé de formule (I) où m est 0 et n est 3.

**5.** Procédé selon la revendication 1 pour préparer un composé de formule (I) où m est 0 et n est 2.

**6.** Procédé selon la revendication 1 pour préparer un composé de formule (I) où m est 0 et n est 4.

**7.** Procédé selon la revendication 4 pour préparer un composé de formule (I) où $R^1$, $R^2$ et $R^3$ sont chacun un hydrogène et $R^4$ est un hydrogène, un alkyle ou un halogène.

**8.** Procédé selon la revendication 1 pour préparer un composé qui est la spiro(dibenzosubérane-5,2'-pyrrolidine).

**9.** Procédé selon la revendication 1 pour préparer un composé qui est la spiro(3-méthyldibenzosubérane-5,2'-pyrrolidine).

**10.** Procédé selon la revendication 1 pour préparer un composé qui est la spiro(3-chlorodibenzosubérane-5,2'-pyrrolidine).

**11.** Procédé pour préparer une composition pharmaceutique comprenant la combinaison d'une quantité pharmacologiquement efficace d'un composé selon la revendication 1, avec un véhicule, diluant et/ou excipient.

**12.** Procédé pour la préparation d'une composition pharmaceutique pour le traitement et la prévention des maladies neurodégénératives, comprenant la combinaison d'un composé tel que défini dans la revendication 1, y compris la spiro(dibenzosubérane-5,3'-pipéridine).

**Revendications pour l'Etat contractant suivant : GR**

**1.** Composé représenté par la formule (I) :

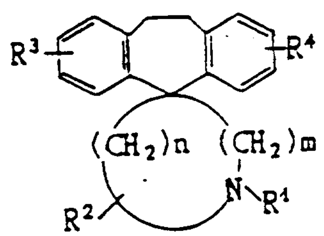

( I )

dans laquelle $R^1$ est un hydrogène, un alkyle, un alcényle ou un aralkyle ; $R^2$ est un hydrogène ou un alkyle ; $R^3$ et $R^4$ qui sont identiques ou différents, sont un hydrogène, un alkyle, un alcoxy ou un halogène ; m est 0 ou 1 ; n est un entier de 1 à 4, ou un sel d'addition d'acide pharmaceutiquement acceptable de celui-ci, à l'exclusion de la spiro(dibenzosubérane-5,3'-pipéridine).

**2.** Composé selon la revendication 1, où m est 0.

**3.** Composé selon la revendication 1, où m est 1.

**4.** Composé selon la revendication 1, où m est 0 et n est 3.

**5.** Composé selon la revendication 1, où m est 0 et n est 2.

**6.** Composé selon la revendication 1, où m est 0 et n est 4.

**7.** Composé selon la revendication 4; où $R^1$, $R^2$ et $R^3$ sont chacun un hydrogène et $R^4$ est un hydrogène, un alkyle ou un halogène.

**8.** Composé selon la revendication 1, qui est la spiro(dibenzosubérane-5,2'-pyrrolidine).

**9.** Composé selon la revendication 1, qui est la spiro(3-méthyldibenzosubérane-5,2'-pyrrolidine).

**10.** Composé selon la revendication 1, qui est la spiro(3-chlorodibenzosubérane-5,2'-pyrrolidine).

**11.** Procédé pour préparer une composition pharmaceutique comprenant la combinaison d'une quantité pharmacologiquement efficace d'un composé selon la revendication 1, avec un véhicule, diluant et/ou excipient.

**12.** Procédé pour préparer un composé de formule I, tel que défini dans l'une quelconque des revendications 1 à 10, qui consiste à

a) soumettre un composé de formule (II) à une réduction

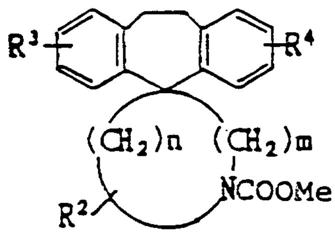

(II)

pour obtenir un composé de formule (I) dans laquelle $R^1$ est Me ; ou

b) soumettre le composé de formule (II) à une hydrolyse puis à une décarboxylation pour obtenir un composé de formule (I) dans laquelle $R^1$ est un hydrogène et, éventuellement, traiter de plus le produit obtenu de façon connue en soi pour introduire un groupe $R^1$ qui est différent de Me.

**13.** Procédé pour la préparation d'une composition pharmaceutique pour le traitement et la prévention des maladies neurodégénératives, comprenant la combinaison d'un composé tel que défini dans la revendication 1, y compris la spiro(dibenzosubérane-5,3'-pipéridine).